# EUROPEAN PATENT SPECIFICATION

(11) **EP 1 146 906 B1**
(45) Date of publication and mention of the grant of the patent: **06.07.2011**
(21) Application number: 00901497.8
(22) Date of filing: 04.02.2000
(51) Int. Cl.: A61K 9/14, A61K 47/02, A61P 37/04, A61P 37/08, A61K 39/35

(54) **MUCOSAL DELIVERY SYSTEM**
MUKOSALES VERABREICHUNGSSYTEM
SYSTEME D'ADMINISTRATION PAR LES MUQUEUSES

(30) Priority: 05.02.1999 DK 15599; 05.02.1999 US 118896 P
(43) Date of publication of application: 24.10.2001
(73) Proprietor: Alk-Abelló A/S, 2970 Hørsholm (DK)
(72) Inventor: IPSEN, Hans, Henrik, DK-3400 Hillerød (DK); ULDAL RAHBEK, Janne, DK-2840 Holte (DK)
(74) Representative: Olsen, Lars Pallisgaard
(86) International application number: PCT/DK2000/000049
(87) International publication number: WO 2000/045847

(56) References cited:
- WO-A-91/16072
- WO-A-94/09823
- FR-A- 2 143 588
- GB-A- 1 078 879
- GB-A- 2 195 996
- US-A- 4 233 288
- BELLIBONI N.: "Oral vaccine and zinc sulfate in the treatment of acne vulgaris double blind trial" FOLHA MEDICA, vol. 82, 1981, pages 459-462, XP000909105
- MC CLURE S. ET AL: "Effects of adjuvants and route of immunization on the intestinal immune response of sheep to ovalbumin" REGIONAL IMMUNOLOGY, vol. 6, 1994, pages 210-217, XP000909090
- MIRCHAMSY H. ET AL: "Stimulating role of toxoid-laden liposomes in oral immunization against diphteria and tetanus infections" BIOLOGICALS, vol. 24, 1996, pages 343-350, XP000909101

## Description

The present invention relates to compositions for the delivery of biologically interactive substances via a mucosal membrane, to vaccines as well as a mucosal delivery system comprising an oxygen-containing metal salt. In other aspects, the present invention relates to uses in generating immune responses, vaccinations and treatments of vertebrates, including human beings, as well as uses in treating, preventing or alleviating allergic reactions. Furthermore, the present invention concerns the use of oxygen-containing metal salts for preparing mucosal delivery systems. Furthermore, the present invention relates to a process for preparing the compositions, the vaccines or the formulations, as well as the products obtainable by the process.

### BACKGROUND OF THE INVENTION

Diseases and various conditions are treated in different ways. Sometimes surgery is the only option, but most conditions are at least at first treated by administering a medicament to the patient curing or alleviating the disease and/or the symptoms. Medicaments are also given as prophylactic treatment.

Sometimes the active compound of a medicament can only be formulated as injectables, or the active compound has little or no effect unless administered parenterally. This is in particular true in the case of many vaccine antigens.

Parenteral treatment must most often be performed by a physician. The appointment requirement is by patients experienced as inconvenient. Furthermore, the injections themselves are usually experienced as unpleasant, all together causing poor patient compliance.

Several parenteral delivery systems are known in the art. They are well described in a large number of textbooks.

Several attempts have been made to prepare mucosal formulations comprising an active compound which is unable to pass the mucosal membrane, thus, avoiding the parenteral route. However, the efforts have not been very successful as evidenced by the very restricted numbers of such products on the market. This is i.a. due to poor bioavailability at the mucosal biomembrane.

### OBJECT OF THE INVENTION

The object of the present invention is to provide a delivery system for delivery of biologically interactive substances via a mucosal membrane of a vertebrate. The mucosal delivery system is useful in the formulation of compositions for delivery of biologically interactive substances via the mucosal membrane. A wide range of other applications making use of the mucosal delivery system and of the compositions is envisaged. In particular, the mucosal delivery system may be incorporated in a vaccine, thus avoiding conventional parenteral vaccination.

Thus, in accordance with the present invention, delivery of biologically interactive substances via the mucosal membrane is possible in an easy, efficient way which should further be acceptable to all patients.

From WO 94/21288 (ref. 1), compositions comprising an admixture of a colloidal metal and an immunologically toxic biologically active factor are known. The toxic biologically factor is selected from cytokines, growth factors and glycoproteins from infectious organisms, e.g. IL-1, IL-2, IL-4, IL-6, IL-8, staphylococcal enterotoxin B, and Macrophage Colony-Stimulating Factor (CSF). The compositions may further comprise an adjuvant such as Freund's complete or incomplete adjuvant, liposomes, muramyl dipeptide or alum. The document further describes a method of administering a toxic biologically-active factor yielding an immunological response to the biologically-active factor while reducing toxic side effects resulting from the biologically-active factor. The composition is preferably administered intravenously, intramuscularly or subcutaneously.

In WO 95/05194 (ref. 2), the use of Hepatitis A virus capsid or a mucosally immunogenic fragment or epitope thereof for the manufacture of a mucosal vaccine composition is described. The mucosal vaccine composition is administered to a mucosal surface of a patient to induce production of serum Immunoglobulin G antibody against Hepatitis A. The composition is formulated for delivery to the nasal or bronchial mucosa.

From JP 65008152 B (ref. 3), oral vaccines are known. The oral vaccines are prepared from bacteria, bacterial toxins, viruses, rickettsia, etc. by (1) inactivation, (2) precipitation by addition of astringents, (3) drying the precipitate and conversion into tablets. Thus, the object is to provide a dry tablet, for which the gastric fluid does not inactivate the product. In the abstract, there is no mentioning of a delivery system ensuring delivery of the immunogenic compound via the mucosal biomembrane.

From GB 2 195 996 (ref. 4), a complex of tea-leaf extract containing the organic compound (-)-epigallocatechin gallate as principal component and active aluminium hydroxide is known. The complexes are stated to possess anti-ulcer activity, anti-peptic and antacid activity. The complex formulated in a composition may suitably be administered orally. Although the composition is intended for oral use, the purpose of this is not delivery of a substance via the mucosal membrane. The target is the interior of the stomach. Thus, mucosal delivery is not disclosed or anticipated.

From GB 1 078 879 (ref. 5), an antacid composition comprising a colloidal aqueous ingestible suspension of aluminium hydroxide and at least one digestive enzyme adsorbed on the aluminium hydroxide is known. The composition is intended for oral use. The composition is for the treatment of stomach upset, indigestion, hyperchlorhydria, peptic ulcer and similar conditions. Although the composition is intended for oral use, the purpose of this is not delivery of a substance via the mucosal membrane. The target is the interior of the stomach. Thus, mucosal delivery is not disclosed or anticipated.

From FR 2 143 588 (ref. 6), a vaccine for immunisation of aquatic birds against goose hepatitis is known. The vaccine may contain aluminium hydroxide as an adjuvant. The vaccine is to be administered by injection. Delivery via a mucosal membrane is not disclosed or anticipated.

### BRIEF DESCRIPTION OF THE FIGURES

Figure 1 shows the IgG1 response on day 35 for mice immunised p.o. with *Tetanus toxoid* suspended with Alhydrogel^{®}.
Figure 2 shows the IgG1 response on day 35 for mice immunised p.o. with *Tetanus toxoid* without Alhydrogel^{®} carrier.
Figure 3 shows the IgG1 response on day 35 for mice immunised i.p. with *Tetanus toxoid* (control group).
Figure 4 shows the IgG2a response on day 35 for mice immunised p.o. with *Tetanus toxoid* suspended with Alhydrogel^{®}.
Figure 5 shows the IgG2a response on day 35 for mice immunised p.o. with *Tetanus toxoid* without Alhydrogel^{®} carrier.
Figure 6 shows the IgG2a response on day 35 for mice immunised i.p. with *Tetanus toxoid* (control group).
Figure 7 shows the IgG1 response on day 56 for mice immunised p.o. with *Tetanus toxoid* suspended with Alhydrogel^{®}.
Figure 8 shows the IgG1 response on day 56 for mice immunised p.o. with *Tetanus toxoid* without Alhydrogel^{®} carrier.
Figure 9 shows the IgG1 response on day 56 for mice immunised i.p. with *Tetanus toxoid* (control group).
Figure 10 shows the IgG2a response on day 56 for mice immunised p.o. with *Tetanus toxoid* suspended with Alhydrogel^{®}.
Figure 11 shows the IgG2a response on day 56 for mice immunised p.o. with *Tetanus toxoid* without Alhydrogel^{®} carrier.
Figure 12 shows the IgG2a response on day 56 for mice immunised i.p. with *Tetanus toxoid* (control group).
Figure 13 shows the IgG1 response on day 70 for mice immunised p.o. with *Tetanus toxoid* suspended with Alhydrogel^{®}.
Figure 14 shows the IgG1 response on day 70 for mice immunised p.o. with *Tetanus toxoid* without Alhydrogel^{®} carrier.
Figure 15 shows the IgG1 response on day 70 for mice immunised i.p. with *Tetanus toxoid* (control group).
Figure 16 shows the IgG2a response on day 70 for mice immunised p.o. with *Tetanus toxoid* suspended with Alhydrogel^{®}.
Figure 17 shows the IgG2a response on day 70 for mice immunised p.o. with *Tetanus toxoid* without Alhydrogel^{®} carrier.
Figure 18 shows the IgG2a response on day 70 for mice immunised i.p. with *Tetanus toxoid* (control group).
Figure 19 shows the IgG1 response on day 70 for mice immunised p.o. with *Phleum pratense* suspended with Alhydrogel^{®}.
Figure 20 shows the IgG1 response on day 70 for mice immunised p.o. with *Phleum pratense* without the Alhydrogel^{®} carrier.
Figure 21 shows the IgG1 response for mice immunised i.p. with *Phleum pratense* (control group).
Figure 22 shows the IgG2a response on day 70 for mice immunised p.o. with *Phleum pratense* suspended with Alhydrogel^{®}.
Figure 23 shows the IgG2a response on day 70 for mice immunised p.o. with *Phleum pratense* without the Alhydrogel^{®} carrier.
Figure 24 shows the IgG2a response for mice immunised i.p. with *Phleum pratense* (control group).
Figure 25 shows the specific Ig response on day 106 for mice immunised s.c. with *Phleum pratense* on day 0, 21, 22, 23.
Figure 26 shows the specific Ig response on day 106 for mice immunised s.c. with *Phleum pratense* on day 0 and p.o. on day 21, 22, 23.
Figure 27 shows the specific Ig response on day 106 for mice immunised s.c. with *Phleum pratense* on day 0 and p.o. with placebo on day 21, 22, 23.

### DETAILED DESCRIPTION OF THE INVENTION

As mentioned above, several attempts have been made to prepare formulations suitable for delivery of substances through the mucosal membrane. The attempts have not been very successful. Either the preparations were not effective, or the efficacies have been reduced, when compared to other routes. Thus, the very limited number of commercially available mucosal delivery formulations on the market indicates the difficulties met when developing such pharmaceutical compositions. It has now surprisingly been found that delivery via the mucosal membrane is in fact possible using a mucosal delivery system as claimed herein.

Thus, in one aspect, the present invention relates to a composition for the delivery of an immunogenic substance via a mucosal membrane of a vertebrate comprising (1) at least one immunogenic substance derived from inhalation allergens or derived from venom allergens, and (2) a mucosal delivery system comprising an oxygen-containing metal salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, barium sulphate and zinc sulphate.

The term "biologically interactive substance" refers to any substance that has a biological effect in itself, or any substance that interacts with or influence other substances, reactions in the body and/or organs resulting in a biological effect. It could also be a substance, that when present in one compartment exerts a biological effect in a different or the same biological compartment.

According to the invention, the biologically interactive substance is an immunogenic substance derived from inhalation allergens or derived from venom allergens.

A wide range of immunogenic substances as well as analogues or derivatives thereof may be applied in the present invention..

Examples of immunogenic substances are allergens, and allergoids.

In the present context, the term analogues or derivatives is intended to include modified forms of the biologically interactive substance. The modification can be made by chemical modification or synthetic modification, e.g. by biotinylation, deamination, maleination, substitution of one or more amino acids, by cross-linking, by glycosylation, or by other recombinant technology. The term is also intended to include natural-occurring mutations, isoforms and retroinverse analogues.

The efficacy of the compositions, formulations and vaccines of the invention is related to the mucosal delivery system, which comprises at least one oxygen-containing metal salt.

The oxygen-containing metal salts can be characterised by a variety of physical-chemical parameters like adsorption, solubility and dissolution properties, ionic charge measured as the isoelectric point pI (pH where the net charge of the substance is zero for a dissociationable compound), dissociation constants, complex coordination, electronic configurations, valence, bonding orbitals and antibonding orbitals, depot properties, adhesion properties, surface characteristics, particle characteristics, and adjuvanticity.

Several features of the metal salts are believed to be important for or connected to the observed beneficial effects of the mucosal delivery system herein described.

Common features for the delivery systems are that they conserve, retain, deliver, and/or enhance the effect of the biological interactive substance in a form available for delivery via the mucosal membrane.

It is believed that the substance of biological value (the biologically interactive substance) is adsorbed (or coupled) to the oxygen-containing metal salt, and this adsorption contributes to the efficacy of the mucosal delivery system, compositions, formulations and vaccines, respectively. Several factors may be important or influence the adsorption between the biological interactive substance and the oxygen-containing metal salt (see e.g. P.M. Callahan et al., Pharmaceutical Research Vol. 8, No. 7, 851-858 (1991) (ref. 7), and Vaccine Design. The Subunit and Adjuvant Approach (ref. 8).

These include pH, the length of time the adsorption reaction is carried out for, mixing conditions, concentrations of the various components in the formulations, compositions and vaccines, containers, temperature, storage, buffer and excipients.

It has been found that in some aspects of the invention the adsorption of the biological interactive substance is influenced by the net/overall charge of the metal salt and the charge of the biological interactive substances, both which are pH dependent.

Metal salt pI may vary (from salt to salt) typically from 2-11 resulting in different optimal coupling pH intervals, Both acidic, alkaline and neutral conditions may be employed, e.g. for commonly used forms of aluminium hydroxide pH 6-10 is used, more acidic intervals are used for aluminium acetate and more alkaline for magnesium hydroxide.

The pI, when relevant, of the dissociationable biological interactive substances may likewise vary within the range of 2-11, e.g., for protein antigens the pI value is often in the range of 4-9.

Thus, adsorption can be carried out over a wide pH range, but preferably conditions are chosen where pH is between the pI of the oxygen-containing metal salt and the average pI of the biological interactive substances to be adsorbed. E.g. effective adsorption has been obtained at pH 8.3, where the pI of the oxygen-containing metal salt, a form of aluminium hydroxide, is 9.2 and the pI of the biological interactive substance, a protein mixture containing *Phleum pratense,* lies within the range of 4-9 for the proteins.

For several of the oxygen-containing metal salts (e.g. Al (OH) ₃, AlPO₄) a high surface area gives a high adsorptive capacity for the biologically interactive substances.

The degree of adsorption will also vary with the metal salt used, and the working concentration of this, e.g. the working concentration may vary for Al³⁺, preferably 0.035-1000 mg/ml formulated as aluminium hydroxide, most preferably 0.35-100 mg/ml.

The working concentration of the biological interactive substance may further influence the adsorption to the metal salt and can vary widely depending on the substance. For proteins, a working concentration range within 0.01 mg/ml-100 mg/ml is preferred, most preferably within 0.01-10 mg/ml.

The interaction between the delivery system and the biological interactive substance is also influenced by the ratio of these, during the adsorption procedure, as well as in the final formulation. The ratio (the amount of biological interactive substance/the amount of mucosal delivery system on a weight basis) should be found in the above-mentioned ranges. These may accordingly vary within 0.01-100,000, preferably within 0.01-100, most preferably within 1-20.

The final formulation should be of such a composition that the amount of the biological substance adsorbed to the oxygen-containing metal salt is in the range of 5-99%, more preferably 10-99% of the added amount.

Further the adsorption has been found to be influenced by the type of buffer system chosen (e.g. NaCl, NaHCO₃, amines), the buffer salt concentration (e.g. 0-40% NaCl) and the presence of excipients (e.g. glycerol 0-70%).

The efficacy of the coupling is dependent on the length of time the reaction is carried out and temperature. Preferably adsorption can be obtained at temperatures between 4-45°C, more preferably between 4-20°C. The preferred length of time for coupling is 0.1-48 hours, more preferably 12-24 hours.

Containers for coupling and storage of the product can be glass and different polymeric materials. It is important to choose a container, which does not adsorb the product. E.g. glass of pharmaceutical grade for proteins.

A further feature believed to be of importance is the solubility of the oxygen-containing metal salts. Preferred oxygen-containing metal salts are water insoluble as well as oxygen-containing metal salts having a water solubility of not more than 0.01 mol/l at 25°C. More preferably insoluble oxygen-containing metal salts or oxygen-containing metal salts with a water solubility of not more than 0.005 mol/l at 25°C.

The oxygen-containing metal salt may further have a depot effect. A depot effect means that the biological interactive substance will be released gradually from the formulation, composition, vaccine or mucosal delivery system. The biological interactive active substance will thus be retained with the oxygen-containing metal salt(s), i.e. with the mucosal delivery system and released gradually therefrom. This is believed to have a number of beneficial effects, e.g. prolonged stimulation, beneficial drug release, and protection of the biological interactive substances against environmental conditions. It is further believed that the oxygen-containing metal salt of the delivery system may possess certain entrapment properties, thus retaining the substance to be delivered via the mucosal membrane.

Another feature of the invention is the protection of the biological interactive substance. Either by maintaining the ideal pH for the biological interactive substance in the microenvironment, thus preventing acid degradation or protecting against enzymatic degradation thereby allowing the substance to be delivered via the mucosal membrane.

Furthermore, some of the oxygen-containing metal salts have a buffer capacity. This may result in an in vivo microenvironment within the delivery system, which protects the biologically interactive substance from the degradable environment. This may e.g. be an advantage in the stomach or intestine where there is a risk of acid and enzymatic degradation, respectively.

A further feature of the oxygen-containing metal salt(s) of the mucosal delivery system can be their capability to adhere to the mucosal membrane, or the effect they may exert on the gastrointestinal movement, e.g. slowing it down. The extended time of passage through the intestine may be beneficial due to the enhanced possibility of prolonging the time allowed for interaction of the biological interactive substance with the target tissue (mucosal membrane), thereby leading to increased transport of the biological interactive substances via the mucosal membrane. Also the muco-adhesive properties of the oxygen-containing metal salt(s)/mucosal delivery system may allow exertion of other functions, e.g., mucosal adjuvant properties, which can result in a faster on-set and effect of the desired response. Different compounds with adjuvant properties are described in e.g. ref. 8, Chapter 7.

It is further believed that the mucosal delivery system of the invention/the oxygen-containing metal salt(s) can be designed to have specific preference for specific mucosal tissues e.g. GALT and Peyers patch, further enhancing the delivery of the active substances at a relevant target site (mucosal tissue) as in EP 266 119 (ref. 9). For several of the oxygen-containing metal salts (e.g. Al(OH)₃, AlPO₄, Ca₃PO₄) the particle size range between 0.5-15 µm.

It has been observed, that an interaction between the biological interactive substances and the immunocompetent tissues in the gastrointestinal tract can take place, resulting in cellular and ultimately humoral systemic responses.

The mucosal delivery system of the invention is for the delivery of an immunogenic substance via a mucosal membrane. Preferred mucosal membranes include the nasal mucosal membrane, the gastrointestinal mucosal membrane, and the sublingual mucosal membrane.

The oxygen-containing metal salt to be used in accordance with the invention may be any oxygen-containing metal salt providing the desired effect when formulated into the mucosal delivery system. Examples of such oxygen-containing substances are aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate, and barium sulphate.

The oxygen-containing metal salts further comprise coordination complexes. A definition of coordination complexes is given in e.g. The Handbook of Chemistry and Physics 56 Ed., Section B, Chapter 7 (1975-76) (ref. 10).

Within the present context, the expression mixed forms is intended to include combinations of the various anions as well as combinations with e.g. chlorides, and sulphides.

Most preferred are aluminium hydroxide, aluminium phosphate, aluminium acetate, calcium phosphate, calcium tartrate and zinc sulphate.

According to the invention the biologically interactive substances is selected from immunogenic substances such as natural, recombinant or modified proteins or fragments thereof, antigens, allergens (cf. below), allergoids such as glutaraldehyde modified allergen complexes, peptides, haptens conjugated on a suitable carrier like KLH (hey hole limpet hemocyanin) carbohydrates, allergoids such as glutaraldehyde modified allergen complexes.

The composition according the invention facilitates delivery via a mucosal membrane anywhere in the body. Thus, the composition can be administered via the oral, nasal, vaginal, sublingual, ocular, rectal, urinal, intramammal, pulmonal, otolar (i.e. via the ear) or buccal route.

The composition may suitably be in the form of a spray, an aerosol, a mixture, tablets (entero- or not-enterocoated), capsule (hard or soft, entero- or not-enterocoated), a suspension, a dispersion, granules, a powder, a solution, an emulsion, chewable tablets, tablets for dissolution, drops, a gel, a paste, a syrup, a cream, a lozenge (powder, granulate, tablets), an instillation fluid, a gas, a vapour, an ointment, a stick, implants (ear, eye, skin, nose, rectal, or vaginal), intramammary preparations, vagitories, suppositories, or uteritories.

In another aspect, the present invention relates to a vaccine for the delivery of an immunogenic substance via a mucosal membrane of a vertebrate comprising (1) at least one immunogenic substance derived from inhalation allergens or derived form venom allergens and (2) a mucosal delivery system comprising an oxygen-containing metal salt salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, barium sulphate and zinc sulphate.

The vaccine may preferably be such wherein the immunogenic substance is selected from natural, recombinant or modified proteins or fragments thereof, allergens, allergoids, haptens, peptides, carbohydrates as well as analogues or derivatives thereof. The vaccine may be in the form of a spray, an aerosol, a mixture, tablets (entero- and not-enterocoated), capsule (hard and soft, entero- and not-enterocoated), a suspension, a dispersion, granules, a powder, a solution, an emulsion, chewable tablets, tablets for dissolution , drops, a gel, a paste, a syrup, a cream, a lozenge (powder, granulate, tablets), an instillation fluid, a gas, a vapour, an ointment, a stick, implants (ear, eye, skin, nose, rectal, and vaginal), intramammary preparations, vagitories, suppositories, or uteritories, suitable for delivery via the oral, nasal, vaginal, sublingual, ocular, rectal, urinal, intramammal, pulmonal, otolar, or buccal route. The vaccine may e.g. be suitable for vaccination, or allergy vaccination (desensitisation), prevention or alleviation of allergic conditions.

A mucosal delivery system for delivery of a biologically interactive substance via a mucosal membrane of a vertebrate also forms part of the present invention. Such delivery system comprises at least one oxygen-containing metal salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate and barium sulphate.

The mucosal delivery system may preferably be in the form of a spray, an aerosol, a mixture, tablets (entero- or not-enterocoated), capsule (hard or soft, entero- or not-enterocoated), a suspension, a dispersion, granules, a powder, a solution, an emulsion, chewable tablets, tablets for dissolution , drops, a gel, a paste, a syrup, a cream, a lozenge (powder, granulate, tablets), an instillation fluid, a gas, a vapour, an ointment, a stick, implants (ear, eye, skin, nose, rectal, or vaginal), intramammary preparations, vagitories, suppositories, or uteritories, suitable for administration via the oral, nasal, vaginal, sublingual, ocular, rectal, urinal, intramammal, pulmonal, otolar, or buccal route.

The present invention also concerns a use of a composition according to the claims for the delivery of a biologically interactive substance via a mucosal membrane of a vertebrate comprising administering a composition as described above to the vertebrate, including a mammal and in particular a human being.

The present invention has a number of important applications.

An example of an important field of applications is the field of vaccination. One of the major achievements of the scientists has been the development of vaccines for preventing life-threatening diseases like e.g. Tetanus or tuberculosis. Vaccination is traditionally performed by the subcutaneous or intramuscular route. By the present invention, vaccination via a mucosal route is indeed possible. In accordance herewith, the present invention relates to a method of generating or altering an immune response in a vertebrate, including a human being, which method comprises administering to the vertebrate a vaccine as defined herein. Furthermore, the invention relates to vaccination or treatment of a vertebrate, including a human being, comprising administering to the vertebrate a vaccine, a formulation, or a composition as defined herein.

In the present context, the term immune response includes specific antibody and/or B- and T-cell activity, the antibodies being from the classes IgA, IgD, IgE, IgG and IgM as well as subclasses thereof. The term is also intended to include other serum or tissue components.

A response may with regard to T-cell activity be regarded as mixed or skewed towards a Th₁ or Th₂ type because of adjuvanticity (ref. 8, Chapter 7). Humorally, it may result in serologic and/or secretory antibodies.

The concept of vaccination is based on two fundamental characteristics of the immune system, namely specificity and memory. Vaccination will prime the immune system of the recipient, and upon repeated exposure to the same proteins the immune system will be in a position to respond more rigorously to the challenge of for example a microbial infection. Vaccines are mixtures of proteins intended to be used in vaccination for the purpose of generating such a protective immune response in the recipient. The protection will comprise only components present in the vaccine and homologous antigens.

An important field of application of the present invention is the field of allergy treatment, including preventing and alleviating allergic reactions. It is well known that genetically predisposed individuals become sensitised (allergic) to antigens originating from a variety of environmental sources, of which the individuals are exposed. The allergic reaction occurs when a previously sensitised individual is re-exposed to the same or a homologous allergen. Allergic responses range from hay fever, rhinoconductivitis, rhinitis and asthma to systemic anaphylaxis and death in response to e.g. bee or hornet sting or insect bite. The reaction is immediate and can be caused by a variety of atopic allergens such as compounds originating from grasses, trees, weeds, insects, (house dust) mites, food, drugs, chemicals and perfumes.

In order to eliminate the strong allergic reactions, carefully controlled and repeated injections with the allergen is commonly used in order to desensitise the patient. Such therapy, where allergic patients are exposed to the offending allergen until a maintenance dose is reached, is usually continued for a long period of time once the maintenance level is reached. This means that specific allergy vaccination include more injections in numbers that a traditional vaccination scheme in healthy people.

Traditionally vaccination is performed by a priming dose, which after a certain period is followed by a boosting. For most vaccines, boosting has to be repeated every 5-10 years in order to maintain the immunological memory intact.

Compared to other types of vaccination, allergy vaccination is complicated by the existence of an ongoing immune response in allergic patients. This immune response is characterised by the presence of allergen-specific IgE, the primary effect of which is the release of allergic symptoms upon exposure to allergens. Thus, allergy vaccination using allergens from natural sources has an inherent risk of side effects being in the utmost consequence life-threatening to the patient.

In accordance with the present invention, uses in the treatment, prevention or alleviation of allergic reactions comprising administering to a vertebrate, including a human being, a vaccine as defined herein are envisaged.

The immunogenic substance may suitably be derived from inhalation allergens originating i.a. from trees, grasses, herbs, fungi, house dust mites, cockroaches and animal hair and dandruff. Important pollen allergens from trees, grasses and herbs are such originating from the taxonomic orders of *Fagales, Oleales* and *Pinales* including i.a. birch *(Betula),* alder *(Alnus),* hazel *(Corylus),* hornbeam *(Carpinus)* and olive *(Olea),* the order of *Poales* including i.a. grasses of the genera *Lolium, Phelum, Poa, Cynodon, Dactylis* and *Secale,* the orders of *Asterales* and *Urticales* including i.a. herbs of the genera *Ambrosia* and *Artemisia.* Important inhalation allergens from fungi are i.a. such originating from the genera *Alternaria* and *Cladosporium.* Other important inhalation allergens are those from house dust mites of the genus *Dermatophagoides,* those from cockroaches and those from mammals such as cat, dog and horse. Further, recombinant allergens according to the invention may be derived from venom allergens including such originating from stinging or biting insects such as those from the taxonomic order of Hymenoptera including bees, wasps, and ants.

It is to be understood that the term derived from includes the naturally-occurring substance as well as isoforms thereof. Furthermore, the substance may be prepared by means of recombinant or synthetic techniques. Specific allergen components are known to the person skilled in the art and include e.g. *Bet v 1 (B. verrucosa,* birch), *Aln g 1 (Alnus glutinosa,* alder), *Cor a* 1 (*Corylus a velana,* hazel) and *Car b* 1 (*Carpinus betulus,* hornbeam) of the *Fagales* order. Others are *Cry j* 1 *(Pinales) , Amb a* 1 and 2, , *Art v* 1 *(Asterales) , Par j* 1 *(Urticales), Ole e* 1 *(Oleales), Ave e* 1, *Cyn d* 1, *Dac g 1, Fes p* 1, *Hol l* 1, *Lol p* 1 and 5, *Pas n* 1, *Phl p* 1 and 5, *Poa p* 1, 2 and 5, *Sec c* 1 and 5, and *Sor h* 1 (various grass pollens), *Alt* a 1 and *Cla h* 1 (fungi), *Der f* 1 and *2, Der p* 1 and 2 (house dust mites, *D. farinae* and *D. pteronyssinus,* respectively), *Bla g* 1 and 2, *Per a* 1 (cockroaches, *Blatella germanica* and *Periplaneta americana,* respectively), *Fel d* 1 (cat), *Can f* 1 (dog), *Equ c* 1, 2 and 3 (horse), *Apis m* 1 and 2 (honeybee), *Ves g* 1, 2 and 5, *Pol a* 1, 2 and 5 (all wasps) and *Sol i* 1, 2, 3 and 4 (fire ant).

Furthermore, the present invention may be useful in veterinary applications. Examples are allergy vaccination or treatment (e.g. fleas, house dust mites. The animals to receive the compositions/formulations of the invention include cats, dogs, birds, poultry, cattle, sheep, horses, other fur-bearing domestic animals, and fish.

It is to be understood that the compositions, formulations, vaccines and mucosal delivery systems of the invention may further comprise adjuvants, excipients or other additives or ingredients suitable for such formulations. Such adjuvants, excipients and other additives or ingredients are well-known to the person skilled in the art, and include i.a. solvents, emulsifiers, wetting agents, plastisizers, colouring substances, fillers, preservatives, viscosity adjusting agents, buffering agents, mucoadhesive and bioadhesive substances. Examples of formulation strategies are well-known to the person skilled in the art. Examples of mucoadhesive and bioadhesive substances are lectins, cellulose or acrylic polymers, chitosan and derivatives, natural gums and polycarylates.

In particular the following adjuvants may be used in the various products of the present invention: interleukins (e.g. IL-1β, IL-2, IL-7, IL-12, Infγ), Adju-Phos^{®}, glucan, antigen formulation, Cholera Holotoxin, liposomes, DDE, DHEA, DMPC, DMPG, DOC/Alum Complex, Freund's incomplete adjuvant, ISCOMs^{®}, LT Oral Adjuvant, muramyl dipeptide, monophosphoryl lipid A, muramyl tripeptide, and phospatidylethanolamine (see also "Vaccine Design. The Subunit and Adjuvant Approach", Chapter 7 (ref. 8)).

Also, administration of the delivery system, the vaccine, or the formulation of the present invention may be combined with other procedures or routes, i.e. administration of the substance parenterally, or by another administration route. The order, sequence or interval of the administration can be varied arbitrarily.

In one embodiment of the invention the delivery system can be used to boost the response to a previously parenteral administered vaccination. The booster doses can preferably be administered (to the immune competent tissue in the nose, mouth, throat, or intestine) via the oral, buccal, sublingual, or nasal route.

The best patient compliance is believed to be obtained with administration via the oral route. The nasal route offers the smallest volumes to be administered, whereas the buccal/sublingual route and the rectal route have intermediate properties. These routes, i.e. oral, nasal, buccal, sublingual and gastrointestinal, are therefore preferred.

The suitable amount of the biologically interactive substance to be administered depends on the substance in question, on the disease or condition to be treated, and further on the age, weight and state of the vertebrate in question. Finding the optimal dose will merely be a matter of routine experimentation to the person skilled in the art.

It is also known to a person skilled in the art, that drugs administered via the mucosa generally have to contain more than 100 times larger doses. Oral administration of birch allergen for vaccination purposes has shown, that the amount, which has to be used in order to obtain a clinical immune therapeutic effect was in 3 orders of magnitude of that used in the parenteral treatment (Taudorf 1992 (ref. 11)). In consistence with other reports, EAACI position paper on local e.g. sublingual immunotherapy also show the use of very large doses (ref. 12). This is also the case for well known peptide drugs, e.g. for the peptide desmopression bioavailability is 1% after oral administration and 10% after nasal administration compared to the parenteral equivalent.

The delivery systems as provided herein allow obtaining efficient response by oral administration with lower amount of biological interactive substances than would be expected from the prior experiments.

The differences in dosing for mucosal versus patenteral route may be expected to vary greatly from substance to substance and route to route. The preferred doses are 0.1-100, most preferably 1-10 times as large as a parenteral bioequivalent dose.

In additional aspect, the present invention relates to a process for preparing a composition, a vaccine or a formulation as described herein by mixing a mucosal delivery system as described herein with a biologically interactive substance, optionally including one or more pharmaceutically acceptable excipients. Thus, the invention also relates to a composition, or a vaccine as described herein obtainable by the process described herein.

An (aqueous) phase containing the biological interactive substance is formed by solution of the substance or by dilution of a more concentrated solution of this with water, optionally containing buffers, salts, solvents or excipients.

An (aqueous) suspension or gel of the oxygen-containing metal salt is formed by adding water, optionally containing buffer, salts, solvents or excipients, to a dry form of the oxygen-containing metal salt or alternatively, optionally adding water containing buffer, salts, excipients, to a pre-equilibrated pre-formed gel. The formulation is optionally made by combining one of these mixtures with a suitable amount of buffer phase, optionally containing salts, solvents, or excipients, and adding the other mixture gradually at a controlled speed under continued stirring or mixing to obtain a concentrated formulation containing a solid phase.

The concentrated formulation is left standing optionally under agitation for a suitable time period.

This may then be filtered off, sedimented or optionally diluted by water, optionally containing buffer, salts, solvents or excipients to yield a final dosing formulation.

Suitable buffers, salts, solvents, or excipients are preferably biologically acceptable, e.g. pharmaceutically acceptable that further are inert or inert amounts with regard to destruction of the structure of the biologically interactive substance or the integrity of the structure of the oxygen-containing metal salt solid phase e.g. gel or the complex between these.

The present invention is further illustrated by the following examples. By the examples, it is further envisaged that the biologically interactive substance is transported via a biomembrane as an immune response is observed. Cellular immune responses are dependent on the one hand on the delivery via the mucosal membrane, and on the other hand on the availability of the substances to the macrophages and/or other antigen presenting cells. Thus, the efficacy of the present invention has been proved.

### EXAMPLES

In the following, some general remarks regarding the experiments are given.

### Animals

Mice were female BALB/c A mice from Bomholtgard, DK-8920 Ry, Denmark, being 6-8 weeks old at the start of the dosing period. They were housed in polycarbonate cages, 8 mice in each cage. Bedding was softwood sawdust. All animals had access to food and water ad libitum. Food was a complete rodent diet from Chr. Petersen A/S, DK-4100 Ringsted, Denmark.

### Animal control groups

Positive control groups: Immunisation by the intraperitoneal route (i.p.) was used as a positive control, as this method is known to induce strong immune responses after a few injections.

### Biologically interactive substances (immunogens):

### Examples 1, and 5:

*Tetanus toxoid (T. toxoid)* (obtained from batches containing either 2.31 or 3.0 mg protein/ml, Statens Serum Institut, DK-2300 Copenhagen S, Denmark). Each dose contained 30 µg *T*. *toxoid* in 0.25 ml.

### Examples 2, 3, and 4:

*P. pratense* (Phl p) pollen extract (obtained from a batch obtained as described below with a protein content of 85.7% w/w, ALK-Abelló A/S, 2970-Hørsholm, Denmark). Each dose contained Phl p extract with a predetermined protein content of 50 µg in 0.25 ml equivalent to 100,000 Standardised Quality units (SQ)/ml.

Preparation of a *Phleum pratense* extract: Pollen from *Phleum pratense* (Allergon AB, Sweden or Biopol Inc., USA) were extracted in 1:10 w/v 0.5 M NaCl for 4 hours under gentle mixing at 3-8°C. The pollen was then removed by centrifugation and subsequent filtration through a 0.22 µm mesh. The clarified extract was then dialysed against distilled water for 72 hours and freeze-dried (cf. also ref. 13).

### Oxygen-containing metal salts:

The molar concentration stated below is for the final formulations.

Aluminium hydroxide, Al(OH)₃ 0.045-0.05 M Al³⁺ (1.25 mg Al³⁺/ml), or 0.04-0.045 M Al³⁺ (1.13 mg Al³⁺/ml) in Example 3. Prepared from Alhydrogel 1.3%^{®} (Superfos, DK-2950 Vedbæk, Denmark).

Calcium phosphate, Ca₃(PO₄)₂ 0.034 M Ca²⁺. Prepared from Calcium Phosphate Adjuvant (Superfos, DK-2950 Vedbæk, Denmark).

Aluminium phosphate, AlPO₄ 0.05 M Al³⁺. Prepared from Adju-phos^{®} (Superfos, DK-2950 Vedbæk, Denmark.

Aluminium acetate, CaH₇O₅Al 0.05 M Al³⁺. Prepared from aluminium acetate, basic (Sigma, USA).

Calcium tartrate, CaC₄H₉O₆ 0.05 M Ca²⁺. Prepared from L-(+)-calcium tartrate hydrate (Sigma, USA).

Zinc sulphate, ZnSO₄ 0.01 M Zn²⁺. Prepared from zinc sulphate, heptahydrate (Sigma USA).

### Preparation of the compositions/formulations/vaccines:

The test formulations/compositions/vaccines were prepared as follows:
The mucosal delivery systems for the delivery of a biologically interactive substance according to the invention comprising an oxygen-containing metal salt were formed as described below.

### Examples 1 and 2:

The immunogen (allergen extract or *Tetanus toxoid*) was dissolved or diluted to a concentration 10 times the concentration of the final formulation. 1/10 vol immunogen solution was mixed with 7/10 vol Coca 0.0 buffer (0.25% sodium hydrogen carbonate and 0.5% sodium chloride). 2/10 vol Alhydrogel^{®} 1.3% was slowly added. In formulations for the control groups, receiving immunogens without Alhydrogel^{®}, the Alhydrogel^{®} was substituted with Coca 0.0 buffer.

These preparations were stored for no more than 3 weeks. Formulations comprising oxygen-containing metal salts and *T. toxoid* were stored at 4°C. All other formulations were stored at -22°C.

### Examples 3, 4, and 5:

The immunogen (allergen extract or *T. toxoid*) was dissolved or diluted to a concentration 10 times the concentration of the final formulation. The metal salt were dissolved or diluted to a concentration five times the concentration of the final formulation with regard to the cation. 1/10 vol immunogen solution was slowly mixed with 2/10 vol metal salt, and left stirring over night at 4°C. The following day 7/10 vol Coca 0.0 buffer (0.25% sodium hydrogen carbonate and 0.5% sodium chloride) was slowly added.

In formulation for the control groups, receiving the metal salt without immunogen, the immunogen was substituted with Coca 0.0. buffer.

In formulations for the control groups, receiving immunogens without metal salt, the metal salt was substituted with Coca 0.0 buffer.

These preparations were stored for no more than 3 weeks. Formulations comprising oxygen-containing metal salts and *T. toxoid* were stored at 4°C. All other formulations were stored at -22°C.

### Dosing:

### Examples 1, 2, 3, and 5:

Peroral (p.o.) administration: The animals were treated for two periods of each 14 days, separated by a 4 weeks period of rest. Thus, according to this, the animals were administered once daily day 0-13, rested day 14-41, and administered once daily day 42-55. The formulation/- vaccine/composition 0.25 ml/dose (i.e. *T. toxoid* or *P. pratense*) were administered by oral gavage (direct delivery to the stomach). The mice were not anaesthetised when dosed.

Intraperitoneally (i.p.) administration: The animals received three doses at 14 days intervals (i.p.), 0.25 ml/dose. The mice were non-anaesthetised when dosed.

### Example 4:

The animals were administered 0.25 ml subcutaneous (s.c.) or 0.25 ml either s.c. or p.o. formulation/vaccine/- composition according to Table 4.

### Blood sampling:

Blood samples were drawn from the orbital vein and sera were analysed individually by ELISA.

### ELISA

Examples 1, 2 and 5 were performed as direct coated ELISA. Examples 3 and 4 were performed as sandwich ELISA.

### Plates:

### Examples 1, 2, 3, 4, and 5:

MaxiSorp^{®} F96, NUNC, Denmark.

All reagents described below were added in a total volume of 100 µl/well.

### Antigen:

### Examples 1 and 5:

*Tetanus toxoid* (*T. toxoid*) 10 µg protein/ml, (obtained from batch containing either 2.31 or 3.0 mg protein/ml, Statens Serum Institut, Copenhagen, Denmark).

### Example 2:

Pollen extract from *Phleum pratense (P. pratense)* 3 µg protein/ml (obtained from a batch obtained as described below with a protein content of 85.7% w/w, ALK-Abelló A/S, Hørsholm, Denmark).

### Examples 3 and 4:

1^{th} layer: 3 µg/ml rabbit a-Ph1 p (ALK-Abelló A/S, Hørsholm, Denmark).
2^{nd} layer: Ph1 p extract 30 µg/ml (obtained from batch with a protein content of 85.7% w/W, ALK-Abelló A/S, Hørsholm, Denmark).

### Positive controls:

### Examples 1 and 5:

Protein A from monoclonal Tetanus IgG2a antibody 5.1G11E5E3 (Statens Serum Institut, Copenhagen, Denmark)

### Examples 2, 3, and 4:

Serum pools from BALB/c A mice immunized i.p. with pollen extract from *P*. *pratense* (Bomholtgard, Ry, Denmark).

### Negative controls:

### Examples 1, and 2:

Serum pools from un-immunised BALB/c A mice 8 month old (Bomholtgård, Ry, Denmark).

### Examples 3, 4, and 5:

Serum pools from un-immunised BALB/c A mice 4 month old (Bomholtgård, Ry, Denmark).

### Secondary antibody:

### Examples 1, and 2:

Biotin anti-mouse IgG1 (Pharmingen, The Netherlands), biotin anti-mouse IgG2a (Pharmingen, The Netherlands), biotin rabbit anti-mouse Ig, P0260 (DAKO, Denmark).

### Examples 3, 4, and 5:

Biotin anti-mouse IgG (Jackson ImmunoResearch Laboratories, USA)

### Substrates:

### Examples 1, and 2:

Peroxidase conjugated streptavidin (DAKO, Denmark).

### Examples 3, 4, and 5:

3,3',5',5 -Tetramethylbenzidine (TMB) (Sigma, USA)

### Immunoreader:

### Examples 1, 2, 3, 4, and 5:

EL 340 Bio Kinetics Reader (Bio-Tek Instruments).

### Analytical procedure:

### Examples 1, and 2:

Plates were coated overnight with the respective antigen. Serum samples from individual animals or control animals were added. The antibody-antigen reaction was allowed to take place overnight. The following day, secondary antibody and substrates were added. The plates were then read in an immunoreader at 470 nm.

### Example 5:

As described under Examples 1, and 2, with the exception that the plates were blocked with bovine serum albumin before serum was added. The plates were read at 450 nm.

### Examples 3, and 4:

Plates were coated overnight with the antibody. The antibody-antigen reaction in 1^{st} and 2^{nd} layer was allowed to take place for one hour. Serum samples were added. This antigen-antibody reaction was allowed to take place for two hours, followed by adding secondary antibody and substrates. The plates were then read in an immunoreader at 470 nm.

### Analysing sera for immunoglobulins:

### Examples 1 and 2:

In order to examine the generated immune response, with regards to Th1/Th2 phenotype, the IgG subclasses were determined. IgG1 was chosen as a marker for a Th2 response, and IgG2a were chosen as a marker for a Th1 response.

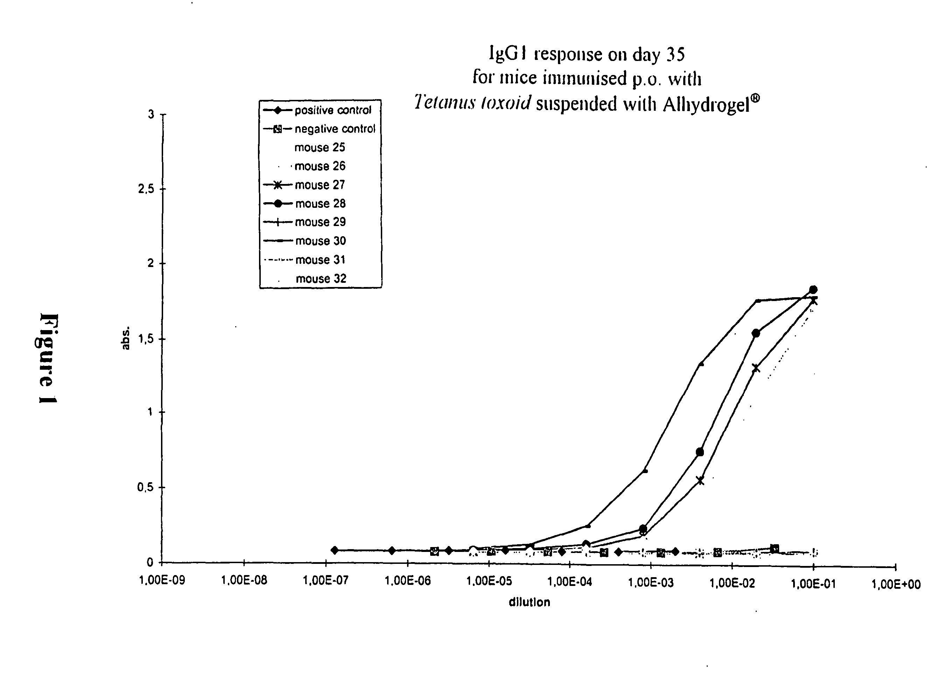

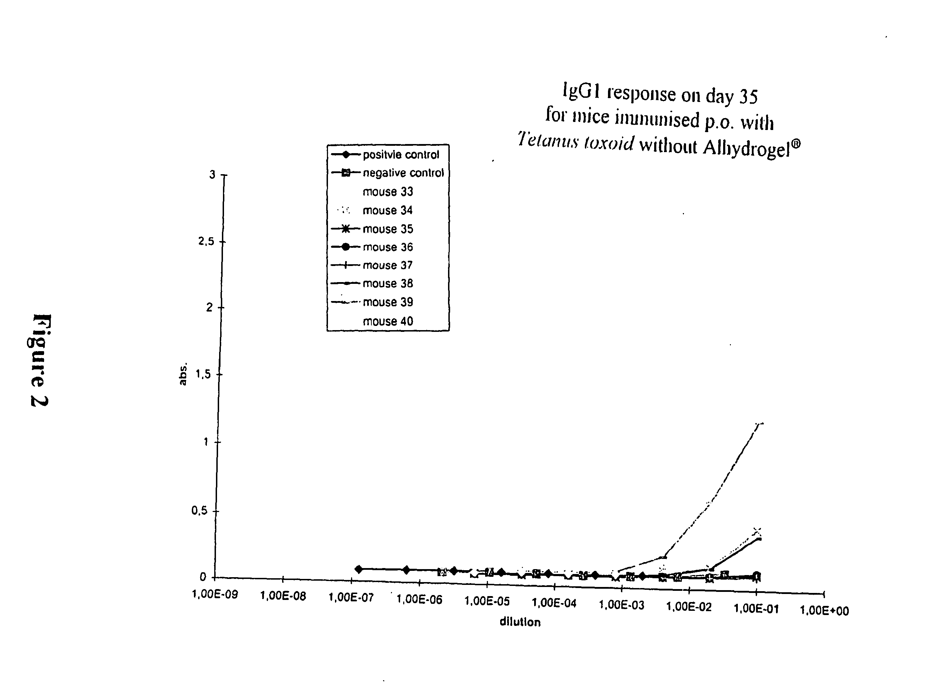

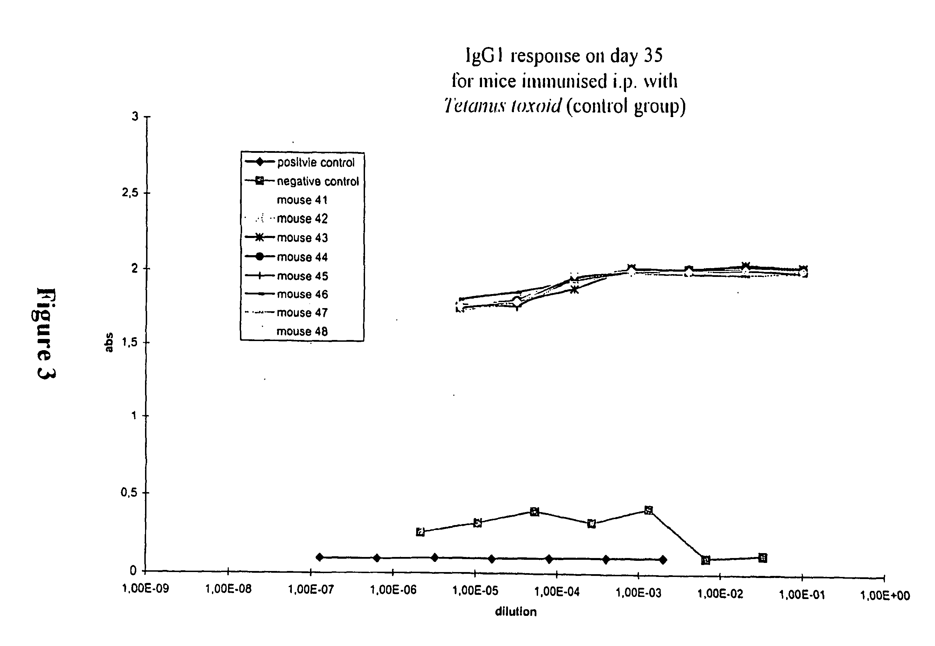

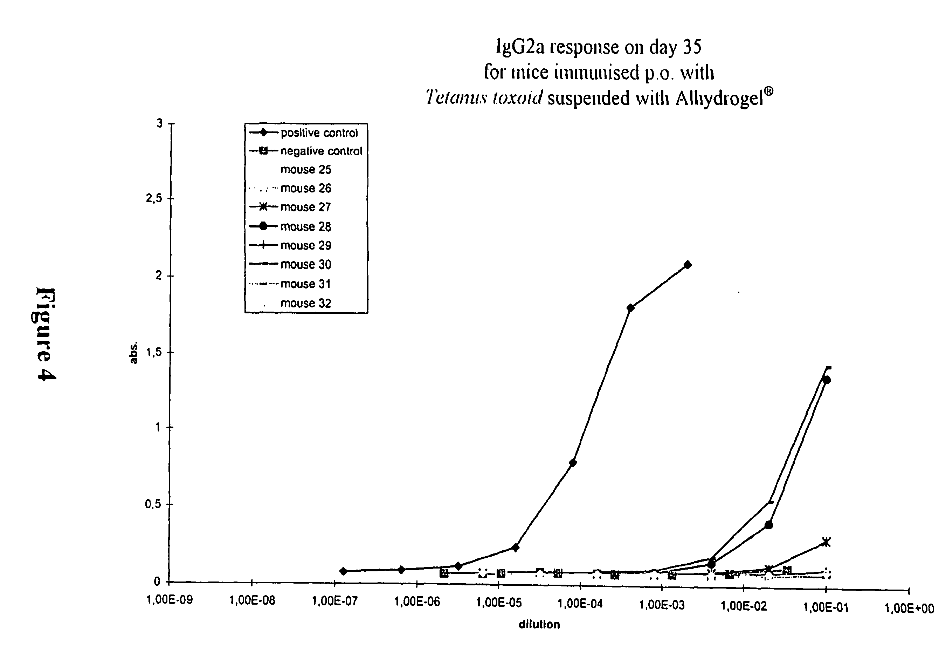

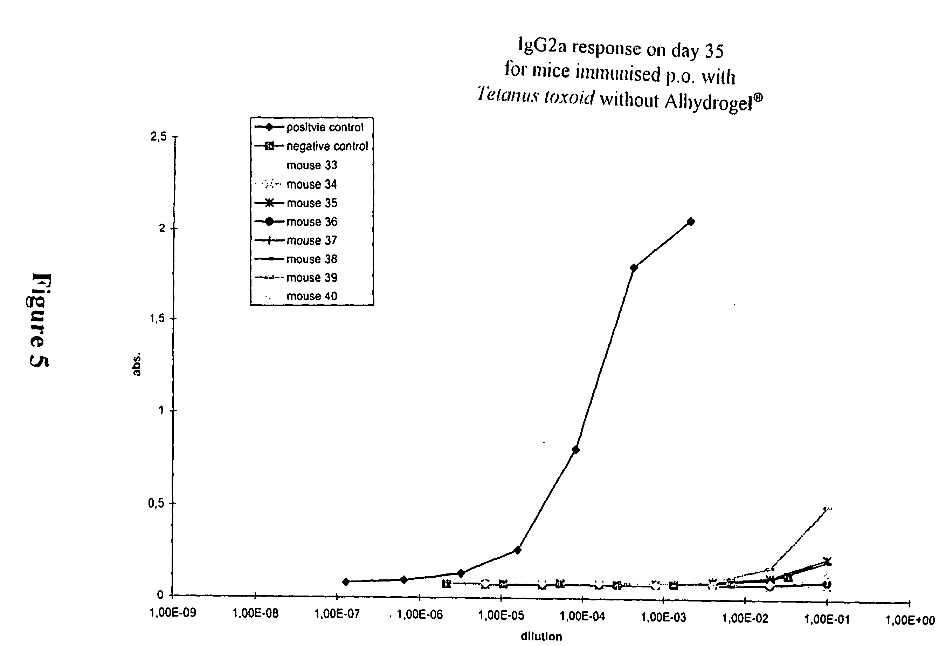

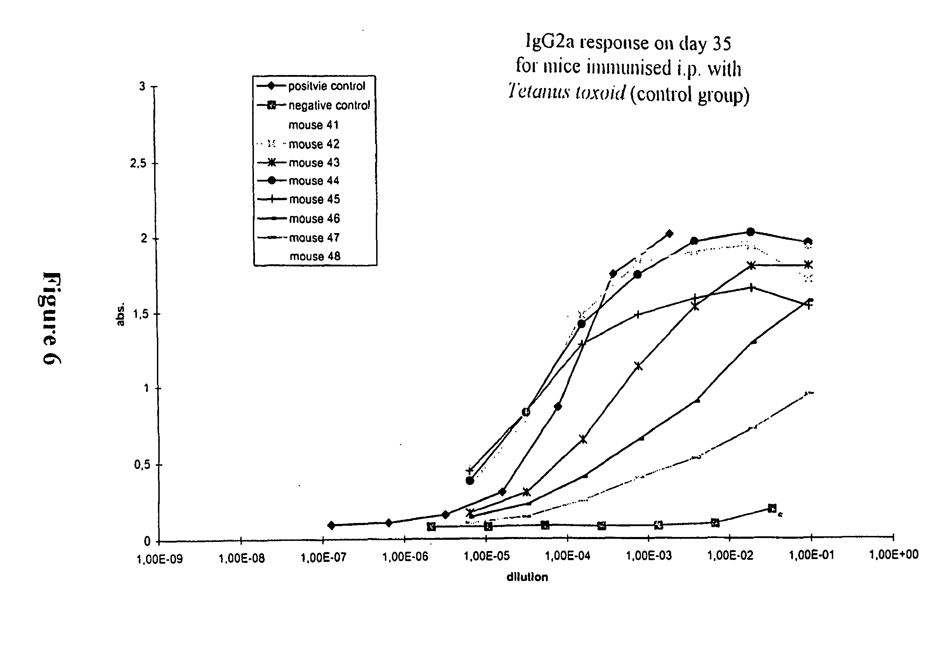

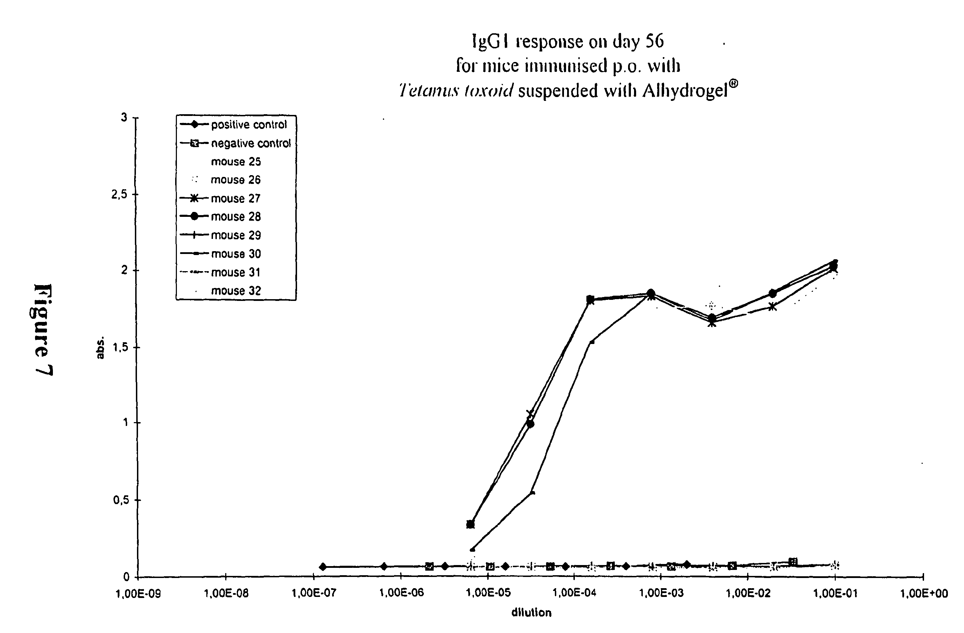

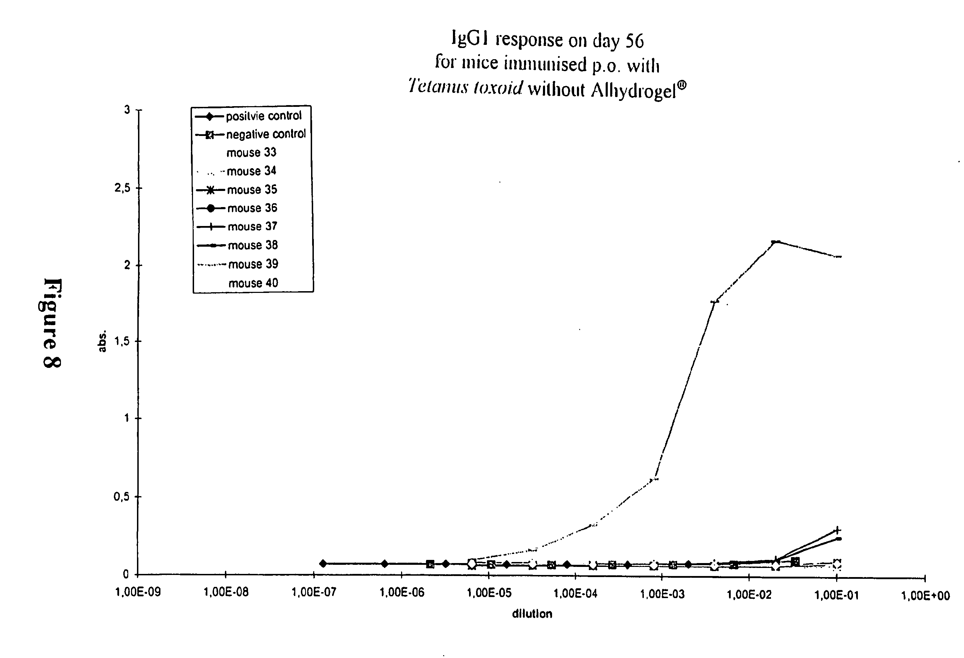

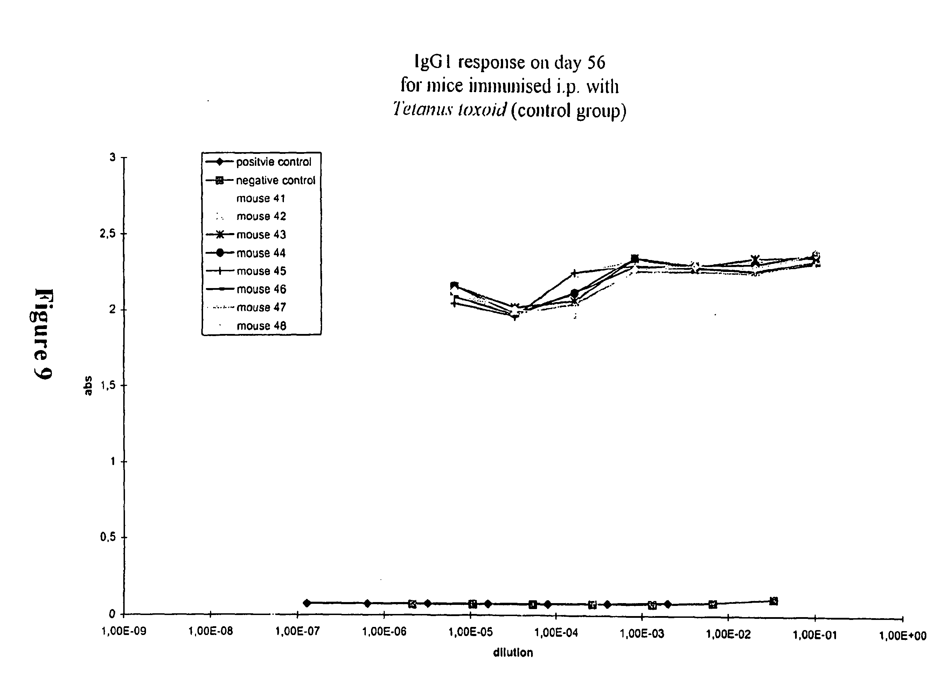

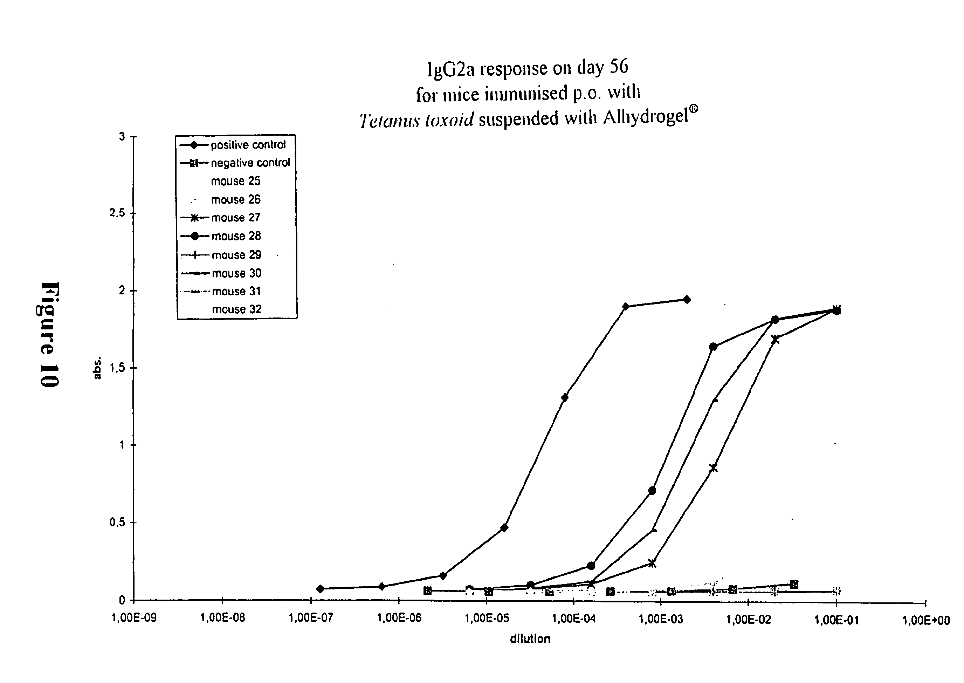

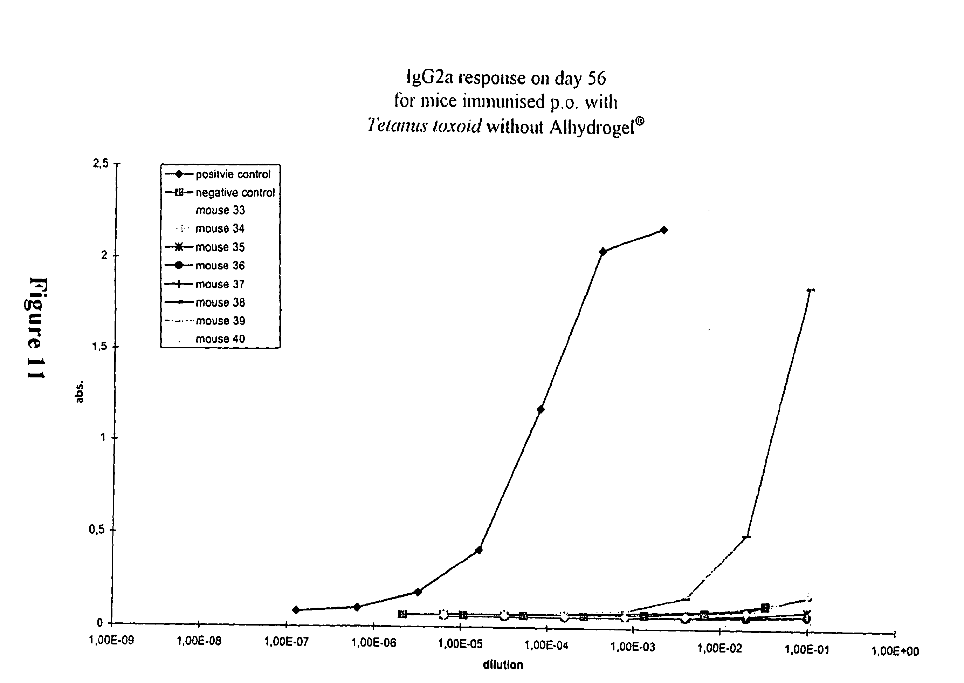

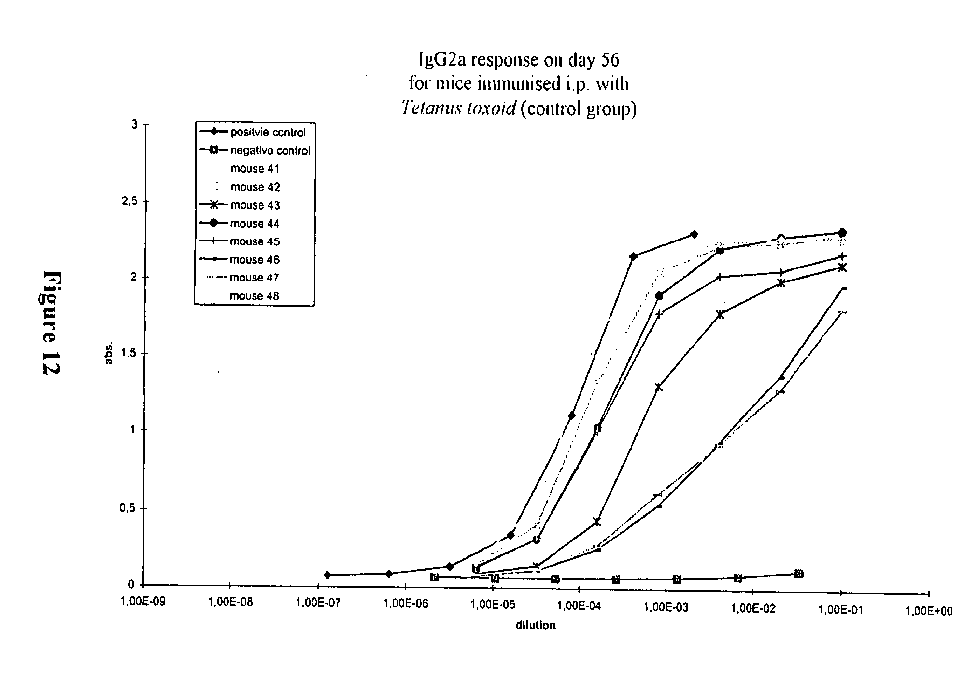

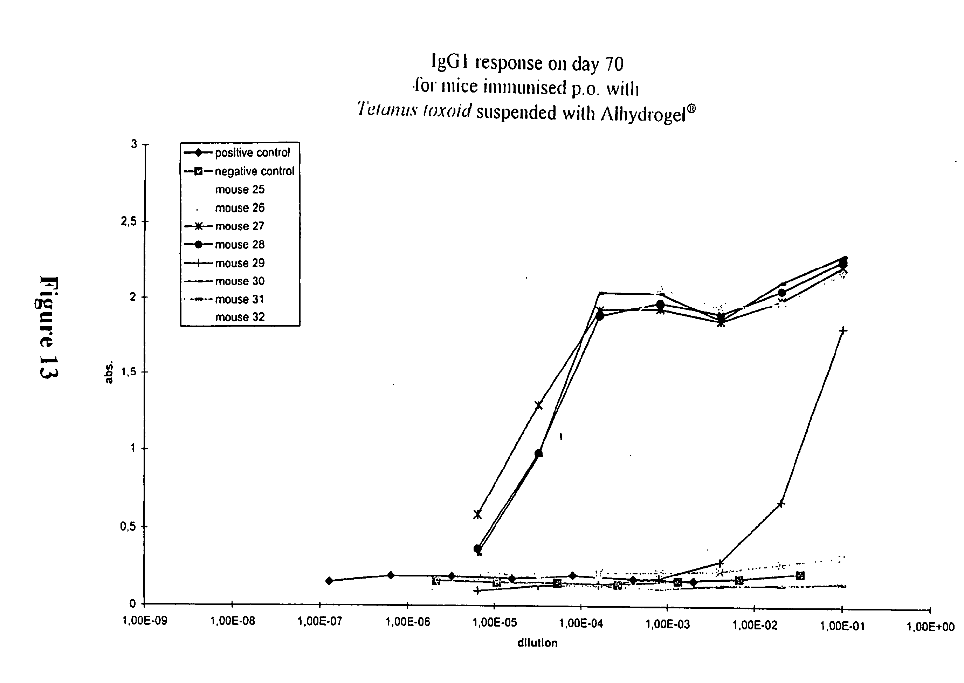

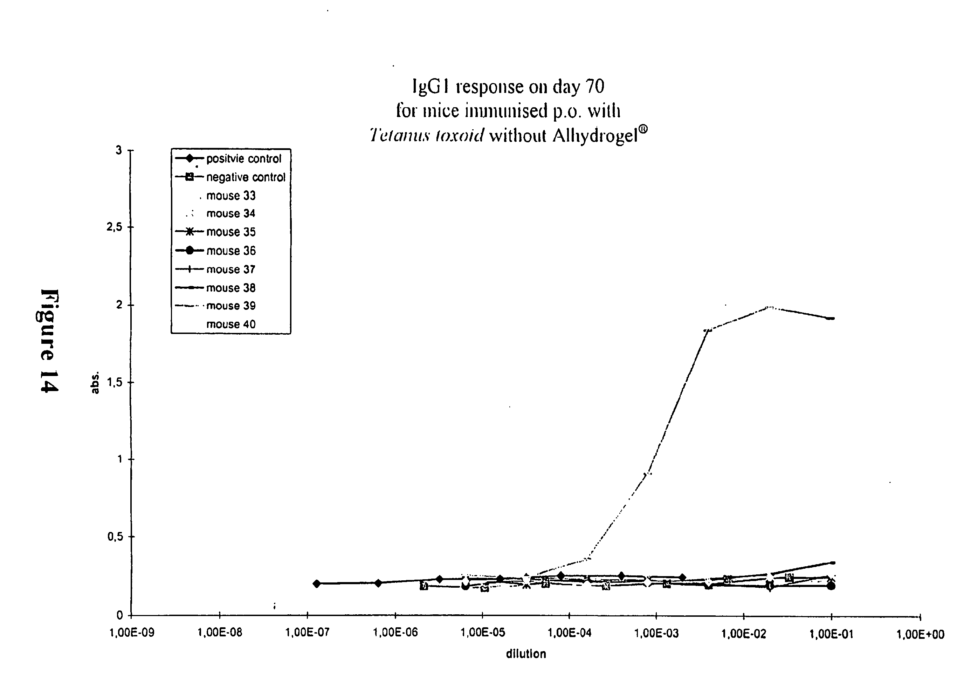

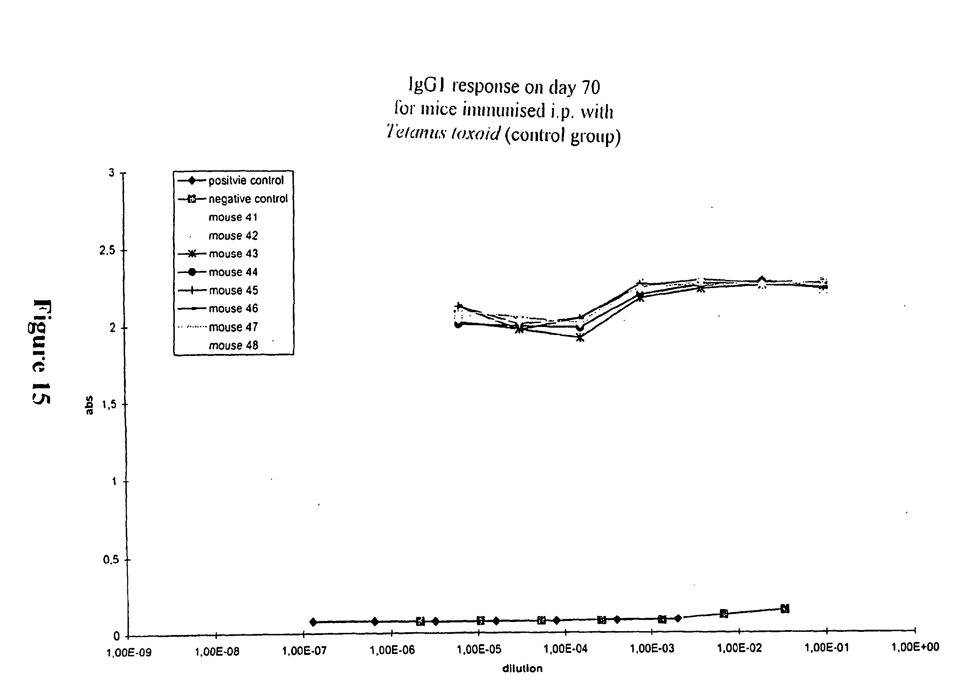

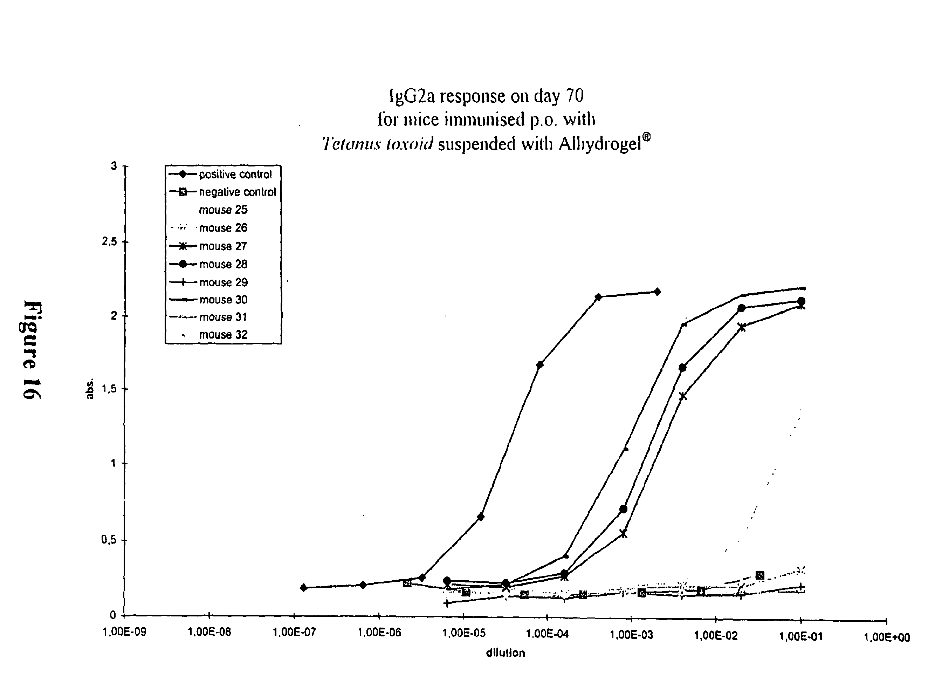

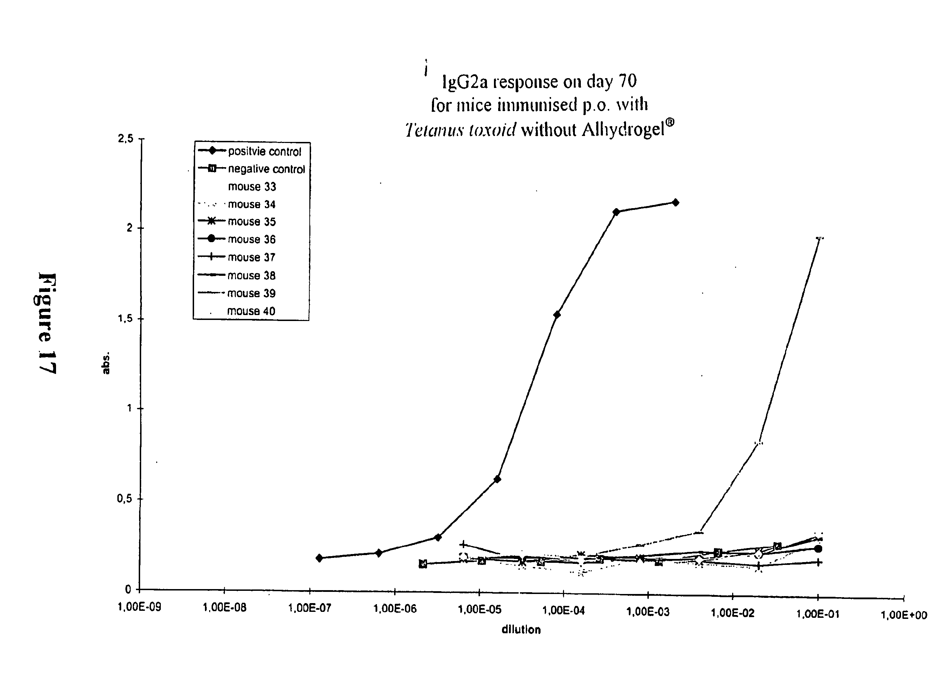

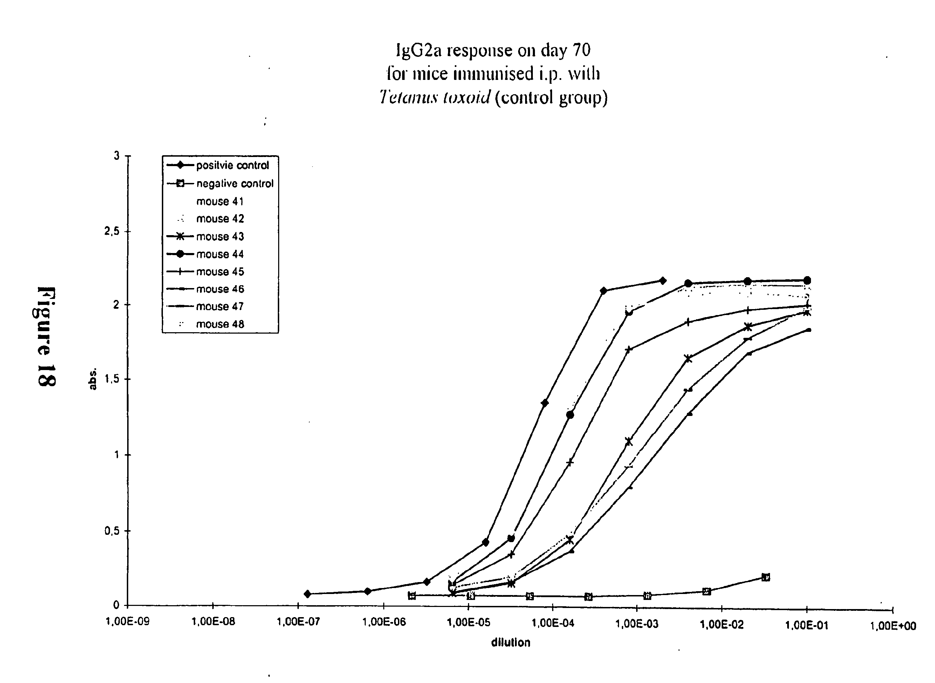

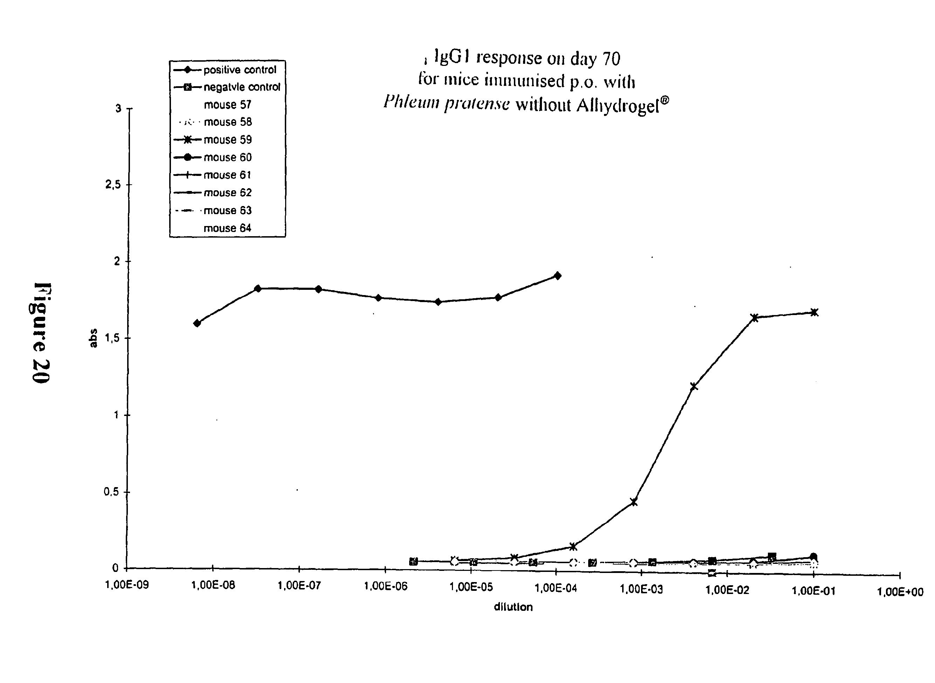

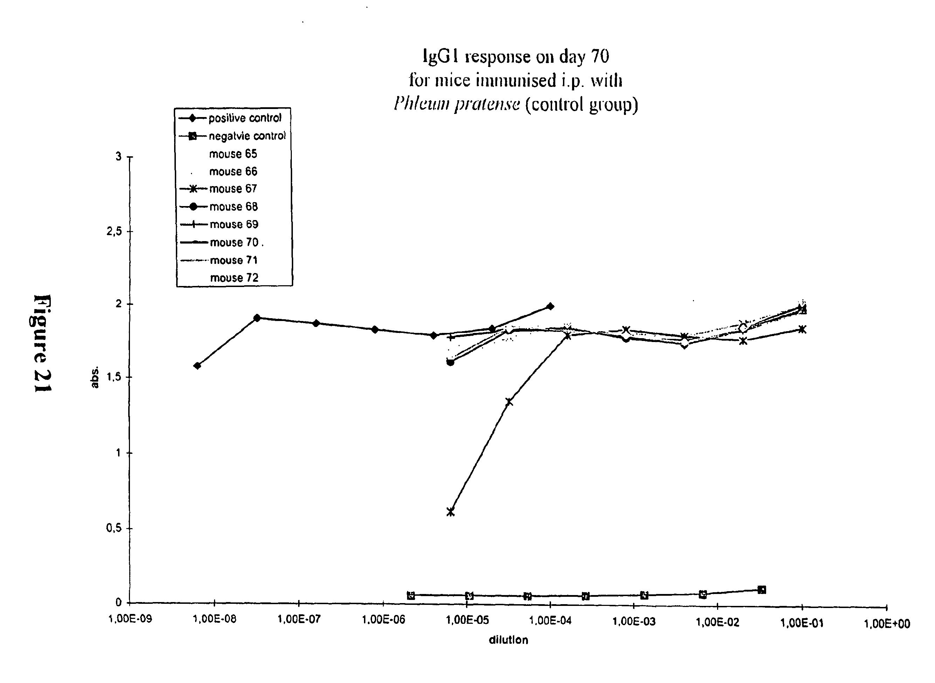

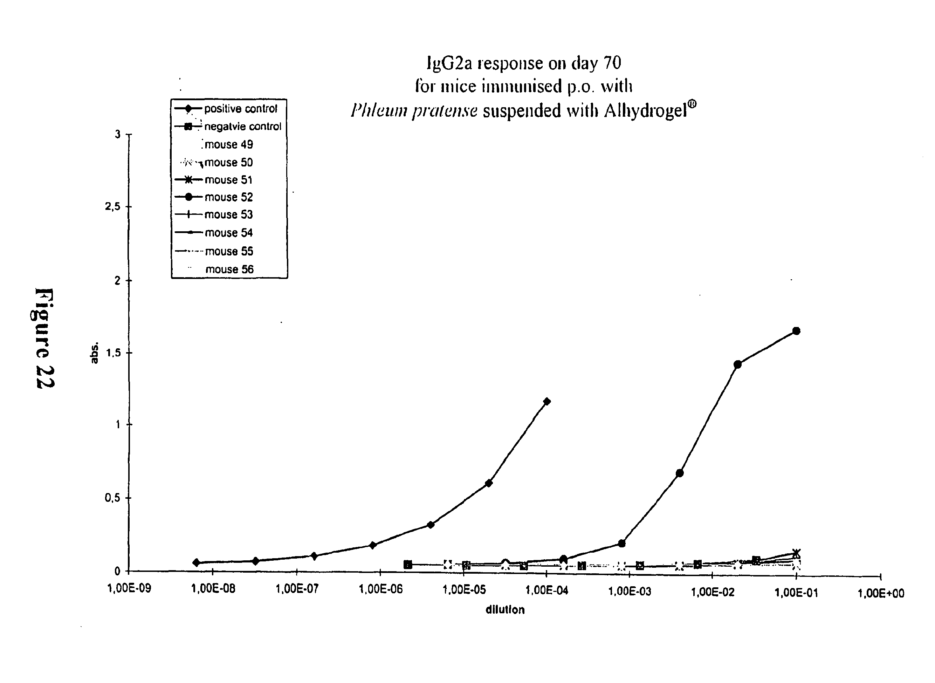

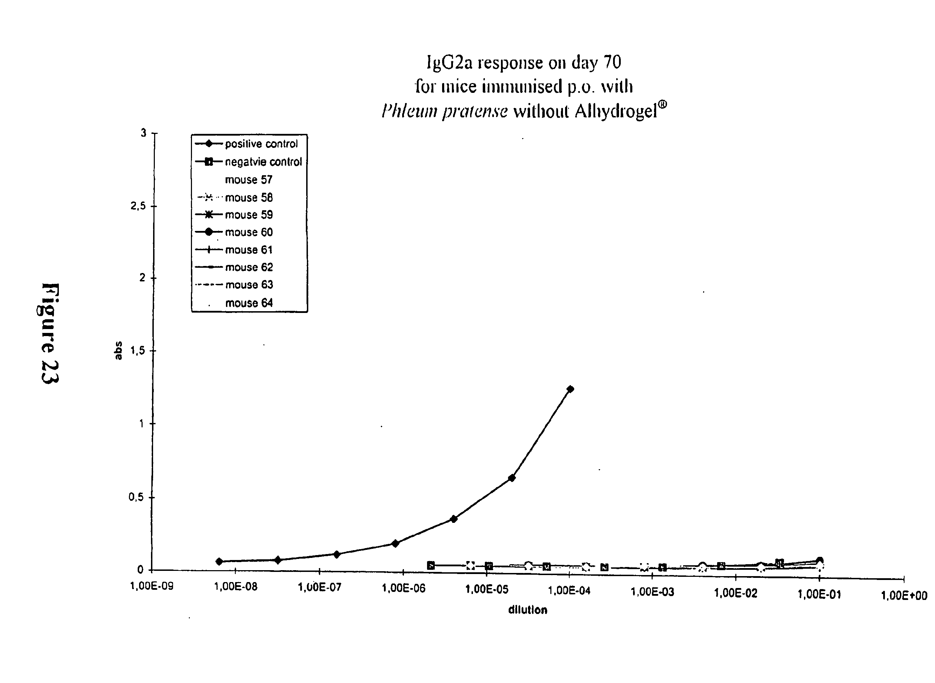

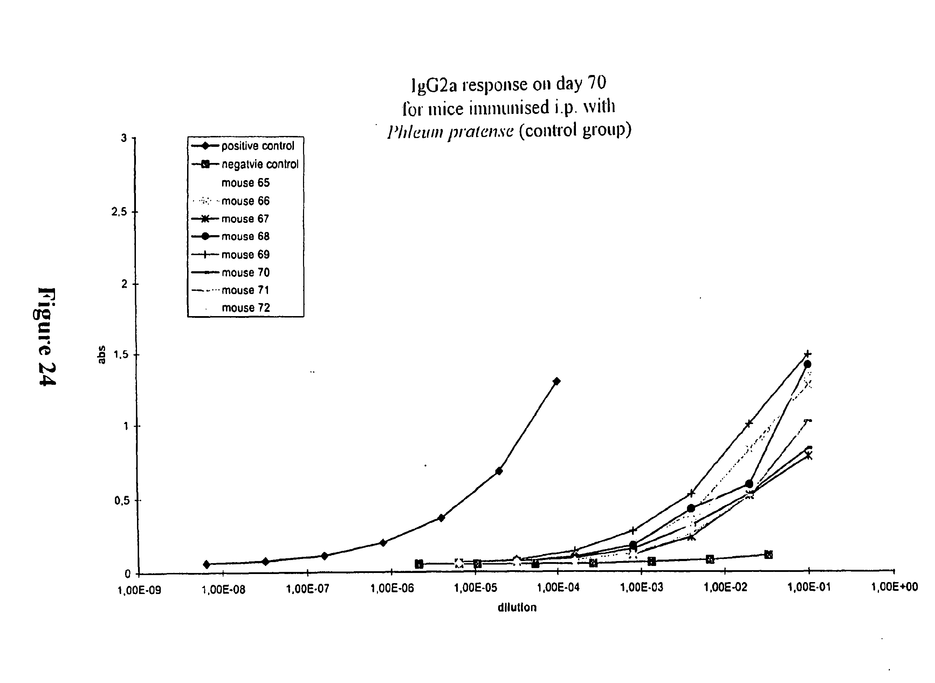

All samples in Examples 1 and 2 were analysed for the presence of both IgG1 and IgG2a. ELISA dilution curves for these experiments are shown in Figures 1-24.

Specific Ig is determined by means of specific anti Ig.

### Example 3, 4, and 5:

### Blood samples were analysed for specific Ig.

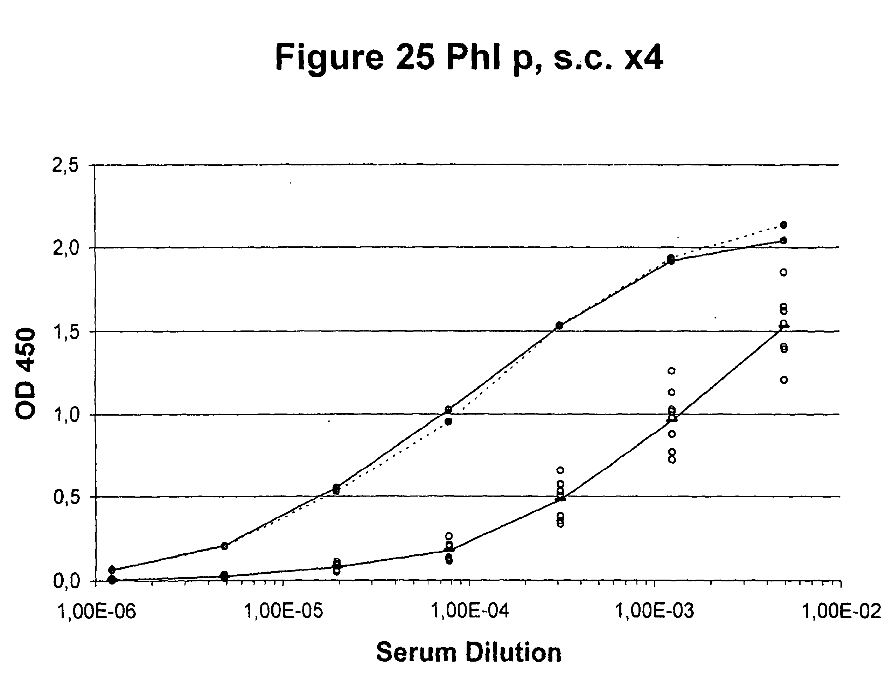

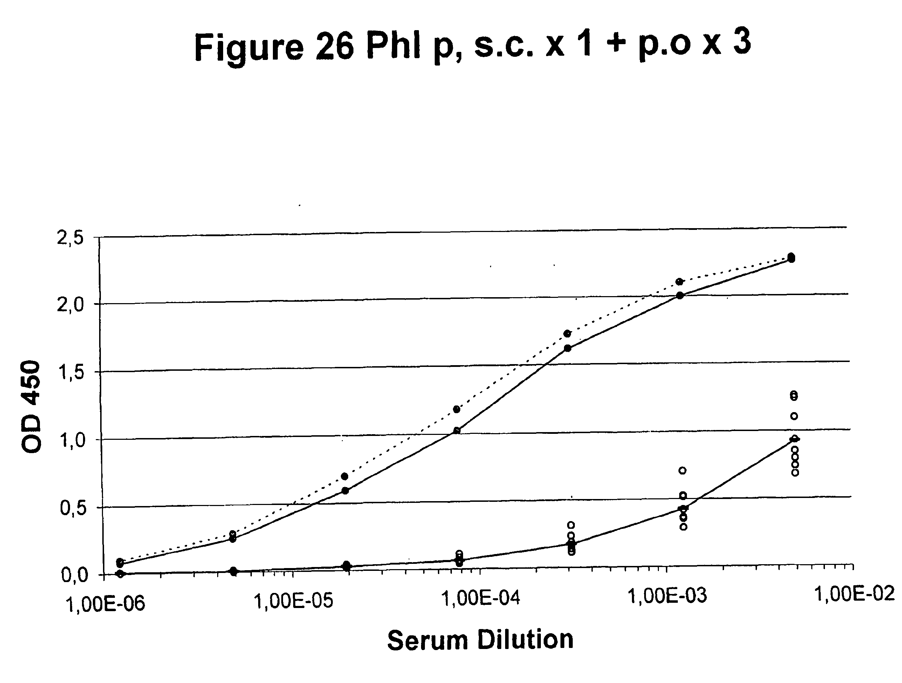

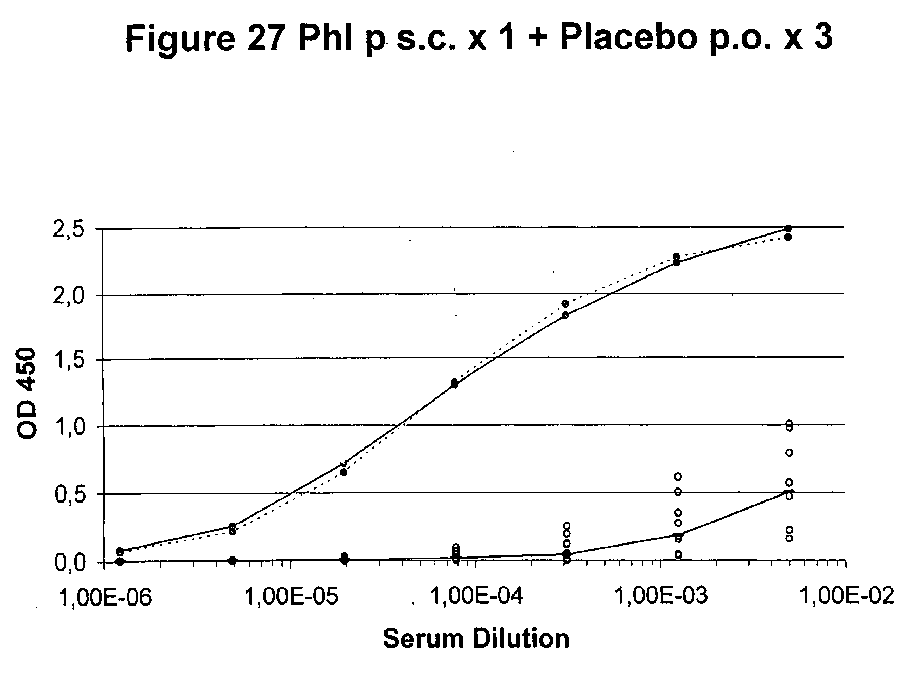

The findings are further illustrated Tables 3-5 and Figures 25-27.

### EXAMPLE 1

### Immunisation with Tetanus toxoid

Immunisation with *T. toxoid* was performed p.o., or i.p. (control animals) as described above. Three test groups of 8 animals each were used. One group received oral doses of the antigen-aluminium hydroxide (0.045-0.05 M) formulation/composition/vaccine prepared as described above. One group received oral doses of the antigen without the oxygen-containing metal salt-containing mucosal delivery system and one group (control animals) was immunised i.p. with the antigen-aluminium hydroxide (0.045-0.05 M) formulation/composition/vaccine as described above. The results obtained are shown in Table 1 below.

Results are indicated as "number of positive responding mice/total number of mice in the group". "Ig tot." indicates specific Ig.

### Positive response

A response is considered positive if the OD-value of the serum is greater than the OD-value of a negative control serum for at least two consecutive dilutions.

The results are depicted in Figures 1-18.

**TABLE 1**

| | P.o. T. toxoid and aluminium hydroxide | | | P.o. T. toxoid without aluminium hydroxide | | | I.p. T. toxoid | | |
|---|---|---|---|---|---|---|---|---|---|
| | Ig | Ig | Ig | Ig | Ig | Ig | Ig | Ig | Ig |
| | tot. | G1 | G2a | tot. | G1 | G2a | tot. | G1 | G2a |
| T. toxoid Day 35 | | 4/8 | 3/8 | | 4/8 | 1/8 | | 8/8 | 8/8 |
| T. toxoid Day 56 | | 5/8 | 4/8 | | 1/8 | 1/8 | | 8/8 | 8/8 |
| T. toxoid Day 70 | 6/8 | 7/8 | 4/8 | 2/8 | 2/8 | 1/8 | 8/8 | 8/8 | 8/8 |

As can be seen from Table 1 and Figures 1-18, the number of positive responding mice (day 70) with levels of specific Ig seen with p.o. immunisation is comparable to the number of positive responding mice observed for i.p. immunisation.

Furthermore, the phenotype of response (IgG1 and IgG2a) indicate both Th₁ and Th₂ responses.

In conclusion, Example 1 shows the feasibility of using the invention for oral vaccination, the immunogenic substance being exemplified by *T. toxoid,* and the mucosal delivery system being exemplified by aluminium hydroxide.

The finding that an immune response is observed, furthermore, indicates that it is possible to use the invention to deliver a biologically interactive substance via the mucosal membrane.

### EXAMPLE 2

### Immunisation with P. pratense

Immunisation was performed with the allergen from *P*. *pratense,* as described above. Three test groups of 8 animals each were used. One group received oral doses of the antigen formulated with aluminium hydroxide-containing mucosal delivery system, prepared as described above. One group received oral doses of the antigen without the oxygen-containing metal salt-containing delivery system, and one group was immunised i.p with the antigen-aluminium hydroxide formulation/vaccine, as described above. The results obtained are shown in Table 2 below. Results are indicated as "number of positive responding mice/total number of mice in the group". "Ig tot." indicates specific Ig.

### Positive response

A response is considered positive if the OD-value of the serum is greater than OD-value of a negative control serum for at least two consecutive dilutions.

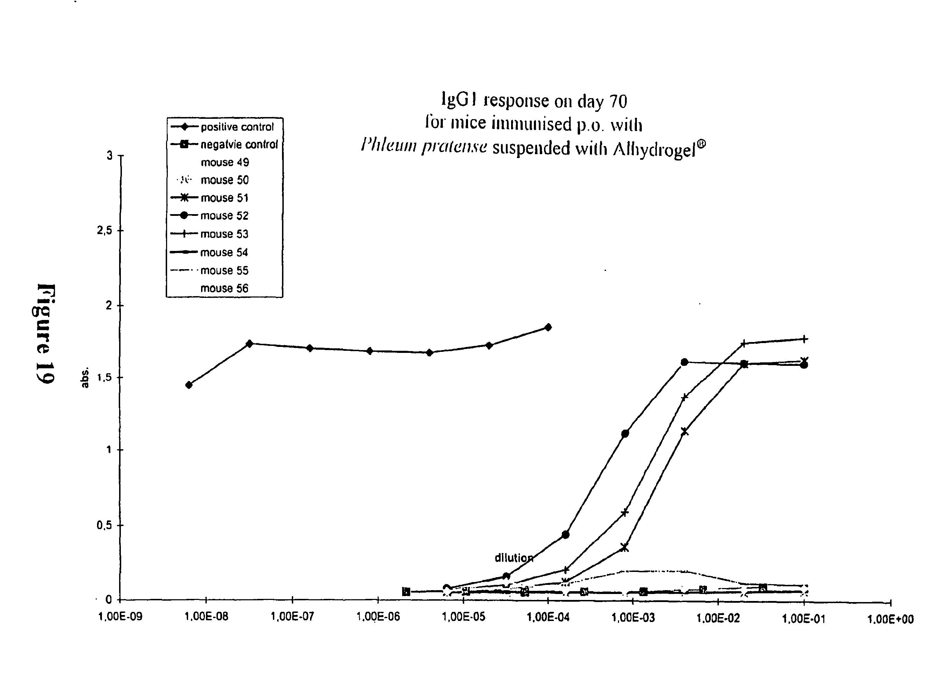

The results are further visualised in Figures 19-24.

**TABLE 2**

| | P.o. Phl p and aluminium hydroxide | | | P.o. Phl p without aluminium hydroxide | | | I.p. Phl 1 | | |
|---|---|---|---|---|---|---|---|---|---|
| | Ig | Ig | Ig | Ig | Ig | Ig | Ig | Ig | Ig |
| | tot. | G1 | G2a | tot. | G1 | G2a | tot. | G1 | G2a |
| Phl p Day 70 | 7/8 | 4/8 | 2/8 | 2/8 | 2/8 | 1/8 | 8/8 | 8/8 | 8/8 |

Table 2 shows the number of mice (day 70) with levels of specific Ig in sera following p.o. immunisation with Ph1 p with or without aluminium hydroxide-containing mucosal delivery system. The number of positively responding mice is comparable to the number of positive responding mice observed following i.p. immunisation.

Furthermore, the type of response (IgG1 and IgG2a) corresponds to that observed with i.p. immunisation. Thus, it is seen that the vaccination in accordance with the invention gives raise to an immunised state similar to that obtained with i.p. vaccination.

In conclusion, Example 2 shows the feasibility of the present invention for allergy vaccination, the immunogenic substance being exemplified by *Phleum pratense,* and the oxygen-containing metal salt being exemplified by aluminium hydroxide.

The results shown in Table 2 furthermore show the uptake via the mucosal membrane of an intact immunogen and subsequent presentation to the relevant cells (B-cells) in a form which retains its three-dimensional structure. Delivery of a biologically interactive substance with an essentially conserved three-dimensional structure is believed to be necessary for a B-cell dependent antibody response. Delivery through the gastrointestinal tract, using other delivery systems, usually induces denaturation of large proteins and peptides and subsequently destroys the three-dimensional structure and the B-cell epitopes, a phenomenon well known for grass allergens (cf. e.g. ref. 11).

### EXAMPLE 3

### Immunisation with Phleum pratense

Immunisation was carried out with Phl p in two different formulations containing an aluminium hydroxide-containing mucosal delivery system. Four test groups, of 8 animals each, were used. One group received Phl p in a formulation containing 1.13 mg Al/ml aluminium hydroxide prepared as described above. One group received Phl p in a formulation containing 1.25 mg Al/ml aluminium hydroxide prepared as described above. Control groups received the same amount of the oxygen-containing metal salt-containing mucosal delivery system formulation, 1.13 mg Al/ml and 1.25 mg Al/ml without allergen in parallel. Results are indicated as "number of positively responding mice/total number of mice in the group".

### Positive response:

A response is considered positive if the OD-value of the serum is greater than the OD-value of a negative control serum for at least two consecutive dilutions.

**TABLE 3**

| Formulation of aluminium hydroxide | Route of adm. | Response |
|---|---|---|
| 1.25 mg/ml + Phl. p. | p.o. | 5/8 |
| Day 70 | | |
| 1.25 mg/ml | p.o. | 1/8 |
| Day 70 | | |
| 1.13 mg/ml + Phl.p. | p.o. | 6/8 |
| Day 70 | | |
| 1.13 mg/ml | p.o. | 1/4 |
| Day 70 | | |

In conclusion, Example 3 shows that various formulations containing different concentrations of the oxygen-containing metal salt can be applied. The concentration of metal salt may be subject to optimisation. In this example, the immunogenic substance is exemplified by *P*. *pratense,* and the oxygen-containing metal salt is exemplified by aluminium hydroxide, used in two different concentrations.

### EXAMPLE 4

### Immunisation with Phleum pratense using different administration protocols

Immunisation was performed with the allergen from *P. pratense.*

Three groups, of 8 mice each, were immunised according to Table 4 and as described under "Dosing" above.

### Positive response:

Sera from groups of mice were analysed individually and the dilution curves obtained are illustrated by a OD-reading for each dilution (Figures 25-27). On top of the points measured, a line illustrating the geometric mean for the group is applied. Two lines obtained from the positive controls are illustrated on each figure due to the fact that a group of eight mice were analysed on two ELISA plates. A response is considered positive if there is a significant difference in the OD response in the same dilution from sera analysed for groups of mice dosed differently.

The results are further visualised in Figures 25-27.

**TABLE 4**

| Group | Route of adm. | Time of dosing | Vaccine formulation |
|---|---|---|---|
| 1 | s.c. | dag 0 | Aluminium |
| s.c.x4 | s.c. | dag 21, 22, 23 | hydroxide and Phl p |
| 2 | s.c. | dag 0 | Aluminium |
| s.c.×1 + | p.o. | dag 21, 22, 23 | hydroxide and |
| p.o.×3 | | | Phl p |
| active | | | |
| 3 | s.c. | dag 0 | Aluminium |
| s.c.×1 + x3 | p.o. | dag 21, 22, 23 | hydroxide |
| placebo | | | without |
| | | | Phl p |
| | | | Placebo |

From comparison of the Figures 25-27 it can be seen that group 2 has a significant higher number of positive responding mice than group 3. Group 1 is included as a positive control and therefore has a significant higher number of positive responding mice than group 2 and 3.

In conclusion, Example 4 shows that by combining different routes of administration it may be possible to induce a strong immune response with active but not with placebo formulations. Furthermore, it may be possible to induce a response that has an earlier debut, higher diagnostic level and/or is very consistent.

The invention may e.g. be used for giving booster immunisations, when applied in combination with conventional vaccination regimes. Alternatively, different administration routes may advantageously be used to improve the vaccination.

In this example, the biologically interactive substance is exemplified by *P. pratense,* and the oxygen-containing metal salt is exemplified by aluminium hydroxide.

### EXAMPLE 5

Immunisation with *Tetanus toxoid* using different oxygen-containing metal salts in the mucosal delivery system The immunisation was performed using *T. toxoid* formulated with different oxygen-containing metal salts (cf. above) as constituent of the mucosal delivery system. 22 groups, each of 8 mice, were immunised according to the protocol described above. 6 different oxygen-containing metal salts were used and the formulations were chosen as molar amount of the cation equivalent to 1.25 mg/ml aluminium hydroxide. All oxygen-containing metal salts were formulated with *T. toxoid,* as described previously. The procedures could probably be further optimised i.a. with regard to reaction times, relative concentrations, and/or pH.

### Positive response:

A response is considered positive if the OD value, measured in sera drawn on day 70 (p.o.) or day 35 (i.p.), is at least 0.5 absorption unit larger than the corresponding signal measured for the negative control group.

**TABLE 5**

| | P.o. T.toxoid | P.o. T.toxoid | P.o. MDs | I.p. T.toxoid |
|---|---|---|---|---|
| | in MDS | without MDS | without T.toxoid | in MDS |
| oxygen-containing metal salt | IgG | IgG | IgG | IgG |
| | Day 70 | Day 70 | Day 70 | Day 35 |
| | | | | |
| Ca₃(PO₄)₂ | 5/7 | 1/8 | 0/8 | 8/8 |
| AlPO₄ | 8/8 | | 0/8 | 8/8 |
| ZnSO₄ | 3/8 | 1/8 | 0/8 | 8/8 |
| Al(OH)₃ | 7/8 | 0/8 | 0/8 | 8/8 |
| CaC₄H₄O₆ | 7/8 | 0/8 | 0/8 | 7/7 |
| Al (OH) (CH₃COO)₂ | 6/8 | | 0/8 | 8/8 |

| | | | | |
|---|---|---|---|---|
| MDS means mucosal delivery system. | | | | |

As can be seen from Table 5 all groups immunised with the antigen formulated to an oxygen-containing metal salt induce an immune response in a larger number of mice than the control groups immunised with either antigen without oxygen-containing metal salt or oxygen-containing metal salt without antigen. All compositions/formulations/- vaccines induce a consistent immune response when used i.p.

In conclusion, Example 5 shows that a wide range of oxygen-containing metal salts can effectively induce an immune response, when used in a mucosal delivery system according to the invention. In this example, the immunogenic substance is exemplified by *T. toxoid* and the metal salt is exemplified by aluminium hydroxide, calcium phosphate, aluminium phosphate, zinc sulphate, calcium tartrate and aluminium acetate, respectively.

### REFERENCES

1. WO 94/21288 (Assay Research, Inc.)
2. WO 95/05194 (Medeva Holdings B.V.)
3. JP 65008152 B (Abstract) (Yaoi, H.)
4. GB 2 195 996 (Mitsui Norin Company Limited)
5. GB 1 078 879 (Parke, Davis & Company)
6. FR 2 143 588 (Herman Schettler)
7. P.M. Callahan et al., Pharmaceutical Research Vol. 8, No. 7, 851-858 (1991)
8. "Vaccine Design. The Subunit and Adjuvant Approach", Chapters 7, 8 and 9, Ed. M.F. Powell & M.J. Newman (1995), Plenum Press
9. EP 266 119 (Southern Research Institute, and The UAB Research Foundation)
10. The Handbook of Chemistry and Physics 56 Ed., Section B, Chapter 7 (1975-1976)
11. E. Taudorf: "Oral Immunotherapy", Doctorate Thesis, 1992, Lægeforeningens Forlag, Copenhagen, Denmark
12. EAACI position paper: "Local immunotherapy", 1998, EAACI Immunotherapy Subcommittee and the ESPACI Immunotherapy Committee
13. Ipsen & Løwenstein, The Journal of Allergy and Clinical Immunology, Vol. 72, No. 2, 150-159 (1983)

## Claims

1. Composition for the delivery of an immunogenic substance via a mucosal membrane of a vertebrate comprising
(1) at least one immunogenic substance derived from inhalation allergens or derived from venom allergens, and
(2) a mucosal delivery system comprising at least one oxygen-containing metal salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, barium sulphate and zinc sulphate.

2. Composition according to claim 1, wherein the oxygen-containing metal salt is aluminium hydroxide, aluminium phosphate, aluminium acetate, calcium phosphate, calcium tartrate or zinc sulphate.

3. Composition according to claims 1-2 wherein the immunogenic substance is derived from inhalation allergens.

4. Composition according to claim 3 wherein the inhalation allergen is originating from trees, grasses, herbs, fungi, house dust mites, cockroaches and animal hair and dandruffs.

5. Composition according to claims 1-4 further comprising a bioadhesive.

6. Composition according to any one of claims 1-5 further comprising a pharmaceutically acceptable adjuvant such as interleukins (e.g. IL-1β, IL-2, IL-7, IL-12, and INFγ), glucan, antigen formulation, Cholera Holotoxin, liposomes, DDE, DHEA, DMPC, DMPG, DOC/Alum Complex, Freund's incomplete adjuvant, LT Oral Adjuvant, muramyl dipeptide, monophosphoryl lipid A, muramyl tripeptide, and phospatidylethanolamine.

7. Composition according to any of claims 1-6 in the form of a spray, an aerosol, a mixture, tablets (entero-or not-enterocoated), capsule (hard or soft, entero- or not-enterocoated), a suspension, a dispersion, granules, a powder, a solution, an emulsion, chewable tablets, tablets for dissolution, drops, a gel, a paste, a syrup, a cream, a lozenge (powder, granulate, tablets), an instillation fluid, a gas, a vapour, an ointment, a stick, implants (ear, eye, skin, nose, rectal, and vaginal), intramammary preparations, vagitories, suppositories, or uteritories.

8. Composition according to any one of claims 1-7 for oral, nasal, vaginal, sublingual, ocular, rectal, urinal, intramammal, pulmonal, otolar (via the ear), or buccal administration.

9. Composition according to any one of claims 1-8, wherein the mucosal membrane is a nasal mucosal membrane, buccal mucosal membrane, a sublingual mucosal membrane, or a gastrointestinal mucosal membrane.

10. Composition according to any of claims 1-9 for use in the treatment, prevention or alleviation of allergic reactions.

11. Vaccine for the delivery of an immunogenic substance via a mucosal membrane of a vertebrate comprising
(1) at least one immunogenic substance derived from inhalation allergens or derived from venom allergens, and
(2) mucosal delivery system comprising at least one oxygen-containing metal salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, barium sulphate and zinc sulphate.

12. Vaccine according to claim 11, wherein the oxygen-containing metal salt is aluminium hydroxide, aluminium phosphate, aluminium acetate, calcium phosphate, calcium tartrate or zinc sulphate.

13. Vaccine according to claims 11-12 wherein the immunogenic substance is derived from inhalation allergens.

14. Vaccine according to claim 13 wherein the inhalation allergen is originating from trees, grasses, herbs, fungi, house dust mites, cockroaches and animal hair and dandruffs.

15. Vaccine according to any one of claims 11-14 further comprising a bioadhesive.

16. Vaccine according to any one of claims 11-15 further comprising a pharmaceutically acceptable adjuvant such as interleukins (e.g. IL-1β, IL-2, IL-7, IL-12, and INFγ), glucan, antigen formulation, Cholera Holotoxin, liposomes, DDE, DHEA, DMPC, DMPG, DOC/Alum Complex, Freund's complete adjuvant, LT Oral Adjuvant, muramyl dipeptide, monophosphoryl lipid A, muramyl tripeptide, and phospatidylethanolamine.

17. Vaccine according to any one of claims 11-16 in the form of a spray, an aerosol, a mixture, tablets (entero-or not-enterocoated), capsule (hard or soft, entero- or not-enterocoated), a suspension, a dispersion, granules, a powder, a solution, an emulsion, chewable tablets, tablets for dissolution, drops, a gel, a paste, a syrup, a cream, a lozenge (powder, granulate, tablets), an instillation fluid, a gas, a vapour, an ointment, a stick, implants (ear, eye, skin, nose, rectal, and vaginal), intramammary preparations, vagitories, suppositories, or uteritories.

18. Vaccine according to any one of claims 11-17 for oral, nasal, vaginal, sublingual, ocular, rectal, urinal, intramammal, pulmonal, otolar, or buccal administration.

19. Vaccine according to any one of claims 11-18, wherein the mucosal membrane is a nasal mucosal membrane, buccal mucosal membrane, a sublingual mucosal membrane, or a gastrointestinal mucosal membrane.

20. Vaccine according to any of the claims 11-19 for use in the treatment, prevention or alleviation of allergic reactions.

21. Use of a mucosal delivery system for the preparation of a medicament comprising a immunogenic substance derived from inhalation allergens or derived from venom allergens for delivery via a mucosal membrane of a vertebrate, wherein said delivery system comprises at least one oxygen-containing metal salt selected from aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, barium sulphate and zinc sulphate.

22. Use according to claims 21, wherein the oxygen-containing metal salt is aluminium hydroxide, aluminium acetate, aluminium phosphate, calcium phosphate, calcium tartrate or zinc sulphate.

23. Use according to claims 21-22 wherein the immunogenic substance is derived from inhalation allergens.

24. Use according to claim 23 wherein the inhalation allergen is originating from trees, grasses, herbs, fungi, house dust mites, cockroaches and animal hair and dandruffs.

25. Use according to claim 21-24, wherein said delivery system further comprises a bioadhesive.

26. Use according to claims 21-25, wherein said delivery system further comprises a pharmaceutically acceptable adjuvant such as interleukins (e.g. IL-1β, IL-2, IL-7, IL-12, and Infγ), , glucan, antigen formulation, Cholera Holotoxin, liposomes, DDE, DHEA, DMPC, DMPG, DOC/Alum Complex, Freund's incomplete adjuvant, LT Oral Adjuvant, muramyl dipeptide, monophosphoryl lipid A, muramyl tripeptide, and phospatidylethanolamine.

27. Use according to claims 21-26, wherein said delivery system is in the form of a spray, an aerosol, a mixture, tablets (entero- or not-enterocoated), capsule (hard and soft, entero- or not-enterocoated), a suspension, a dispersion, granules, a powder, a solution, an emulsion, chewable tablets, tablets for dissolution, drops, a gel, a paste, a syrup, a cream, a lozenge (powder, granulate, tablets), an instillation fluid, a gas, a vapour, an ointment, a stick, implants (ear, eye, skin, nose, rectal, or vaginal), intramammary preparations, vagitories, suppositories, or uteritories.

28. Use according to claims 21-27, wherein said delivery system is formulated for oral, nasal, vaginal, sublingual, ocular, rectal, urinal, intramammal, pulmonal, otolar, or buccal administration.

29. Use according to claims 21-28, wherein the mucosal membrane is a nasal mucosal membrane, buccal mucosal membrane, a sublingual mucosal membrane, or a gastrointestinal mucosal membrane.

30. Use according to claims 21-29 for the preparation of a medicament for treatment, prevention or alleviation of allergic reactions.

31. Use of a composition according to claims 1-10 for the manufacture of a medicament for the treatment, prevention or alleviation of allergic reactions.

32. Use of a vaccine according to claims 11-20 for the manufacture of a medicament for the treatment, prevention or alleviation of allergic reactions.

33. Use according to claims 31-32 for the manufacture of a medicament in a form for delivery via a buccal, sublingual, nasal or intestinal membrane.

34. Use according to claim 31 wherein a composition according to claims 1-10 is used for the manufacture of the vaccine.

35. Use according to claims 31-34 for the manufacture of a vaccine for specific allergy vaccination treatment or prevention.

## Patentansprüche

1. Zusammensetzung zur Verabreichung einer immunogenen Substanz über eine mukosale Membran eines Vertebraten, umfassend
(1) mindestens eine aus Inhalationsallergenen stammende oder aus Giftallergenen stammende immunogene Substanz und
(2) ein mukosales Verabreichungssystem, umfassend mindestens ein sauerstoffenthaltendes Metallsalz, das aus Aluminiumhydroxid, Aluminiumphosphat, Aluminiumsulfat, Aluminiumazetat, Kaliumaluminiumsulfat, Kalziumphosphat, Kalziumtartrat, Maalox (Mischung des Aluminiumhydroxids und Magnesiumhydroxids), Berylliumhydroxid, Zinkhydroxid, Zinkcarbonat, Bariumsulfat und Zinksulfat ausgewählt ist.

2. Zusammensetzung nach Anspruch 1, wobei das sauerstoffenthaltende Metallsalz ein Aluminiumhydroxid, Aluminiumphosphat, Aluminiumazetat, Kalziumphosphat, Kalziumtartrat oder Zinksulfat ist.

3. Zusammensetzung nach den Ansprüchen 1-2, wobei die immunogene Substanz aus Inhalationsallergenen stammt.

4. Zusammensetzung nach Anspruch 3, wobei das Inhalationsallergen von Bäumen, Gräsern, Kräutern, Pilzen, Hausstaubmilben, Kakerlaken und Tierhaar sowie Schuppen abstammt.

5. Zusammensetzung nach den Ansprüchen 1-4, zusätzlich umfassend ein bioadhäsives Mittel.

6. Zusammensetzung nach irgendeinem der Ansprüche 1-5, zusätzlich umfassend ein pharmazeutisch annehmbares Adjuvans wie etwa Interleukine (zum Beispiel IL-1β, IL-2, IL-7, IL-12 und INFγ), Glucan, Antigenformulierung, Cholera-Holotoxin, Liposome, DDE, DHEA, DMPC, DMPG, DOC/Alum-Komplex, Freunds unvollständiges Adjuvans, orales Adjuvans LT, Muramyldipeptid, Monophosphoryllipid A, Muramyltripeptid und Phosphatidylethanolamin.

7. Zusammensetzung nach irgendeinem der Ansprüche 1-6 in Form eines Sprays, eines Aerosols, einer Mischung, Tabletten (darm- oder nicht-darmbeschichtet), einer Kapsel (hart oder weich, darm- oder nicht-darmbeschichtet), einer Suspension, einer Dispersion, Granalien, eines Pulvers, einer Lösung, einer Emulsion, kaubarer Tabletten, Tabletten für Auflösung, Tropfen, eines Gels, einer Paste, eines Sirups, einer Creme, einer Pastille (Pulver, Granulat, Tabletten), eines Instillationsfluids, eines Gases, eines Dampfs, einer Salbe, eines Stifts, Implantate (Ohr, Auge, Haut, Nase, rektal und vaginal), intramammärer Vorbereitungen, Vagitorien, Suppositorien oder Uteritorien.

8. Zusammensetzung nach irgendeinem der Ansprüche 1-7 zur oralen, nasalen, vaginalen, sublingualen, okularen, rektalen, urinalen, intramammalen, pulmonalen, otolaren (durch das Ohr) oder bukkalen Eingabe.

9. Zusammensetzung nach irgendeinem der Ansprüche 1-8, wobei die mukosale Membran eine nasale mukosale Membran, bukkale mukosale Membran, eine sublinguale mukosale Membran oder eine gastrointestinale mukosale Membran ist.

10. Zusammensetzung nach irgendeinem der Ansprüche 1-9 zur Verwendung bei der Behandlung, Vorbeugung oder Linderung von allergischen Reaktionen.

11. Vakzin zur Verabreichung einer immunogenen Substanz über eine mukosale Membran eines Vertebraten, umfassend
(1) mindestens eine aus Inhalationsallergenen stammende oder aus Giftallergenen stammende immunogene Substanz und
(2) ein mukosales Verabreichungssystem, umfassend mindestens ein sauerstoffenthaltendes Metallsalz, das aus Aluminiumhydroxid, Aluminiumphosphat, Aluminiumsulfat, Aluminiumazetat, Kaliumaluminiumsulfat, Kalziumphosphat, Kalziumtartrat, Maalox (Mischung des Aluminiumhydroxids und Magnesiumhydroxids), Berylliumhydroxid, Zinkhydroxid, Zinkcarbonat, Bariumsulfat und Zinksulfat ausgewählt ist.

12. Vakzin nach Anspruch 11, wobei das sauerstoffenthaltende Metallsalz ein Aluminiumhydroxid, Aluminiumphosphat, Aluminiumazetat, Kalziumphosphat, Kalziumtartrat oder Zinksulfat ist.

13. Vakzin nach den Ansprüchen 11-12, wobei die immunogene Substanz aus Inhalationsallergenen stammt.

14. Vakzin nach Anspruch 13, wobei das Inhalationsallergen von Bäumen, Gräsern, Kräutern, Pilzen, Hausstaubmilben, Kakerlaken und Tierhaar sowie Schuppen abstammt.

15. Vakzin nach irgendeinem der Ansprüche 11-14, zusätzlich umfassend ein bioadhäsives Mittel.

16. Vakzin nach irgendeinem der Ansprüche 11-15, zusätzlich umfassend ein pharmazeutisch annehmbares Adjuvans wie etwa Interleukine (zum Beispiel IL-1β, IL-2, IL-7, IL-12 und INFγ), Glucan, Antigenformulierung, Cholera-Holotoxin, Liposome, DDE, DHEA, DMPC, DMPG, DOC/Alum-Komplex, Freunds vollständiges Adjuvans, orales Adjuvans LT, Muramyldipeptid, Monophosphoryllipid A, Muramyltripeptid und Phosphatidylethanolamin.

17. Vakzin nach irgendeinem der Ansprüche 11-16 in Form eines Sprays, eines Aerosols, einer Mischung, Tabletten (darm- oder nicht-darmbeschichtet), einer Kapsel (hart oder weich, darm- oder nicht-darmbeschichtet), einer Suspension, einer Dispersion, Granalien, eines Pulvers, einer Lösung, einer Emulsion, kaubarer Tabletten, Tabletten für Auflösung, Tropfen, eines Gels, einer Paste, eines Sirups, einer Creme, einer Pastille (Pulver, Granulat, Tabletten), eines Instillationsfluids, eines Gases, eines Dampfs, einer Salbe, eines Stifts, Implantate (Ohr, Auge, Haut, Nase, rektal und vaginal), intramammärer Vorbereitungen, Vagitorien, Suppositorien oder Uteritorien.

18. Vakzin nach irgendeinem der Ansprüche 11-17 zur oralen, nasalen, vaginalen, sublingualen, okularen, rektalen, urinalen, intramammalen, pulmonalen, otolaren oder bukkalen Eingabe.

19. Vakzin nach irgendeinem der Ansprüche 11-18, wobei die mukosale Membran eine nasale mukosale Membran, bukkale mukosale Membran, eine sublinguale mukosale Membran oder eine gastrointestinale mukosale Membran ist.

20. Vakzin nach irgendeinem der Ansprüche 11-19 zur Verwendung bei der Behandlung, Vorbeugung oder Linderung von allergischen Reaktionen.

21. Verwendung eines mukosalen Verabreichungssystems zur Vorbereitung eines Medikaments, umfassend eine aus Inhalationsallergenen stammende oder aus Giftallergenen stammende immunogene Substanz zur Verabreichung über eine mukosale Membran eines Vertebraten, wobei das Verabreichungssystem mindestens ein sauerstoffenthaltendes Metallsalz umfasst, das aus Aluminiumhydroxid, Aluminiumphosphat, Aluminiumsulfat, Aluminiumazetat, Kaliumaluminiumsulfat, Kalziumphosphat, Kalziumtartrat, Maalox (Mischung des Aluminiumhydroxids und Magnesiumhydroxids), Berylliumhydroxid, Zinkhydroxid, Zinkcarbonat, Bariumsulfat und Zinksulfat ausgewählt ist.

22. Verwendung nach Anspruch 21, wobei das sauerstoffenthaltende Metallsalz ein Aluminiumhydroxid, Aluminiumazetat, Aluminiumphosphat, Kalziumphosphat, Kalziumtartrat oder Zinksulfat ist.

23. Verwendung nach den Ansprüchen 21-22, wobei die immunogene Substanz aus Inhalationsallergenen stammt.

24. Verwendung nach Anspruch 23, wobei das Inhalationsallergen von Bäumen, Gräsern, Kräutern, Pilzen, Hausstaubmilben, Kakerlaken und Tierhaar sowie Schuppen abstammt.

25. Verwendung nach den Ansprüchen 21-24, wobei das Verabreichungssystem zusätzlich ein bioadhäsives Mittel umfasst.

26. Verwendung nach den Ansprüchen 21-25, wobei das Verabreichungssystem zusätzlich ein pharmazeutisch annehmbares Adjuvans wie etwa Interleukine (zum Beispiel IL-1ß, IL-2, IL-7, IL-12 und INFγ), Glucan, Antigenformulierung, Cholera-Holotoxin, Liposome, DDE, DHEA, DMPC, DMPG, DOC/Alum-Komplex, Freunds unvollständiges Adjuvans, orales Adjuvans LT, Muramyldipeptid, Monophosphoryllipid A, Muramyltripeptid und Phosphatidylethanolamin umfasst.

27. Verwendung nach den Ansprüchen 21-26, wobei das Verabreichungssystem in Form eines Sprays, eines Aerosols, einer Mischung, Tabletten (darm- oder nicht-darmbeschichtet), einer Kapsel (hart und weich, darm-oder nicht-darmbeschichtet), einer Suspension, einer Dispersion, Granalien, eines Pulvers, einer Lösung, einer Emulsion, kaubarer Tabletten, Tabletten für Auflösung, Tropfen, eines Gels, einer Paste, eines Sirups, einer Creme, einer Pastille (Pulver, Granulat, Tabletten), eines Instillationsfluids, eines Gases, eines Dampfs, einer Salbe, eines Stifts, Implantate (Ohr, Auge, Haut, Nase, rektal oder vaginal), intramammärer Vorbereitungen, Vagitorien, Suppositorien oder Uteritorien ist.

28. Verwendung nach den Ansprüchen 21-27, wobei das Verabreichungssystem zur oralen, nasalen, vaginalen, sublingualen, okularen, rektalen, urinalen, intramammalen, pulmonalen, otolaren oder bukkalen Eingabe formuliert ist.

29. Verwendung nach den Ansprüchen 21-28, wobei die mukosale Membran eine nasale mukosale Membran, bukkale mukosale Membran, eine sublinguale mukosale Membran oder eine gastrointestinale mukosale Membran ist.

30. Verwendung nach den Ansprüchen 21-29 zur Vorbereitung eines Medikaments zur Behandlung, Vorbeugung oder Linderung von allergischen Reaktionen.

31. Verwendung einer Zusammensetzung nach den Ansprüchen 1-10 zur Herstellung eines Medikaments zur Behandlung, Vorbeugung oder Linderung von allergischen Reaktionen.

32. Verwendung eines Vakzins nach den Ansprüchen 11-20 zur Herstellung eines Medikaments zur Behandlung, Vorbeugung oder Linderung von allergischen Reaktionen.

33. Verwendung nach den Ansprüchen 31-32 zur Herstellung eines Medikaments in einer Form zur Verabreichung über eine bukkale, sublinguale, nasale oder intestinale Membran.

34. Verwendung nach Anspruch 31, wobei eine Zusammensetzung nach den Ansprüchen 1-10 zur Herstellung des Vakzins verwendet wird.

35. Verwendung nach den Ansprüchen 31-34 zur Herstellung eines Vakzins zur spezifischen Allergie-Vakzination-Behandlung oder -Vorbeugung.

## Revendications

1. Composition pour l'administration d'une substance immunogène par l'intermédiaire d'une membrane muqueuse d'une vertèbre, comprenant
(1) au moins une substance immunogène dérivée d'allergènes d'inhalation ou dérivée d'allergènes de venin, et
(2) un système d'administration muqueuse comprenant au moins un sel métallique oxygéné sélectionné parmi le groupe composé de l'hydroxyde d'aluminium, du phosphate d'aluminium, du sulfate d'aluminium, de l'acétate d'aluminium, du sulfate d'aluminium de potassium, du phosphate de calcium, du tartrate de calcium, du Maalox (mélange d'hydroxyde d'aluminium et d'hydroxyde de magnésium), de l'hydroxyde de béryllium, de l'hydroxyde de zinc, du carbonate de zinc, du sulfate de baryum et du sulfate de zinc.

2. Composition selon la revendication 1, dans laquelle le sel métallique oxygéné est l'hydroxyde d'aluminium, le phosphate d'aluminium, l'acétate d'aluminium, le phosphate de calcium, le tartrate de calcium ou le sulfate de zinc.

3. Composition selon les revendications 1 à 2, dans laquelle la substance immunogène est dérivée d'allergènes d'inhalation.

4. Composition selon la revendication 3, dans laquelle l'allergène d'inhalation dérive de bois, herbes, verdures, champignons, acariens de poussière de maison, cafards, poils d'animaux et écailles.

5. Composition selon les revendications 1 à 4, comportant en outre un bioadhésif.

6. Composition selon l'une quelconque des revendications 1 à 5, comportant en outre un adjuvant pharmaceutiquement acceptable tel que les interleukines (p. ex. Il-1β, IL-2, IL-7, IL-12 et INFγ), glucane, formulation d'antigène, l'holotoxine du choléra, liposomes, DDE, DHEA, DMPC, DMPG, DOC/Alum Complexe, l'adjuvant incomplet de Freund, adjuvant oral LT, muramyldipeptide, monophosphoryl lipide A, muramyltripeptide et phosphatidyléthanolamine.

7. Composition selon l'une quelconque des revendications 1 à 6 dans la forme d'un atomiseur, un aérosol, un mélange, des comprimés (à enrobage entérosoluble ou non entérosoluble), capsule (dur ou mou, à enrobage entérosoluble ou non entérosoluble), une suspension, une dispersion, granules, une poudre, une solution, une émulsion, comprimés à croquer, comprimés pour la dissolution, gouttes, un gel, une pâte, un sirop, une crème, une pastille (poudre, granulés, comprimés), un fluide d'instillation, un gaz, une vapeur, une pommade, un poussoir, implants (oreille, oeil, peau, nez, rectaux et vaginaux), préparations intramammaires, vagitoires, suppositoires ou utérines.

8. Composition selon l'une quelconque des revendications 1 à 7 pour l'administration orale, nasale, vaginale, sublinguale, oculaire, rectale, d'urinal, intramammaire, pulmonaire, auriculaire (par l'intermédiaire de l'oreille) ou buccale.

9. Composition selon l'une quelconque des revendications 1 à 8, dans laquelle la membrane muqueuse est une membrane muqueuse nasale, membrane muqueuse buccale, une membrane muqueuse sublinguale ou une membrane muqueuse gastro-intestinale.

10. Composition selon l'une quelconque des revendications 1 à 9 pour l'usage dans le traitement, la prévention ou l'allégement des réactions allergiques.

11. Vaccin pour l'administration d'une substance immunogène par l'intermédiaire d'une membrane muqueuse d'une vertèbre, comprenant
(1) au moins une substance immunogène dérivée d'allergènes d'inhalation ou dérivée d'allergènes de venin, et
(2) un système d'administration muqueuse comprenant au moins un sel métallique oxygéné sélectionné parmi le groupe composé de l'hydroxyde d'aluminium, du phosphate d'aluminium, du sulfate d'aluminium, de l'acétate d'aluminium, du sulfate d'aluminium de potassium, du phosphate de calcium, du tartrate de calcium, du Maalox (mélange d'hydroxyde d'aluminium et d'hydroxyde de magnésium), de l'hydroxyde de béryllium, de l'hydroxyde de zinc, du carbonate de zinc, du sulfate de baryum et du sulfate de zinc.

12. Vaccin selon la revendication 11, dans lequel le sel métallique oxygéné est l'hydroxyde d'aluminium, le phosphate d'aluminium, l'acétate d'aluminium, le phosphate de calcium, le tartrate de calcium ou le sulfate de zinc.

13. Vaccin selon les revendications 11 à 12, dans lequel la substance immunogène est dérivée d'allergènes d'inhalation.

14. Vaccin selon la revendication 13, dans lequel l'allergène d'inhalation dérive de bois, herbes, verdures, champignons, acariens de poussière de maison, cafards, poils d'animaux et écailles.

15. Vaccin selon l'une quelconque des revendications 11 à 14, comportant en outre un bioadhésif.

16. Vaccin selon l'une quelconque des revendications 11 à 15, comportant en outre un adjuvant pharmaceutiquement acceptable tel que les interleukines (p. ex. IL-1β, IL-2, IL-7, IL-12, et INFγ), glucane, formulation d'antigène, l'holotoxine du choléra, liposomes, DDE, DHEA, DMPC, DMPG, DOC/Alum Complexe, l'adjuvant complet de Freund, adjuvant oral LT, muramyldipeptide, monophosphoryl lipide A, muramyltripeptide et phosphatidyléthanolamine.

17. Vaccin selon l'une quelconque des revendications 11 à 16 dans la forme d'un atomiseur, un aérosol, un mélange, des comprimés (à enrobage entérosoluble ou non entérosoluble), capsule (dur ou mou, à enrobage entérosoluble ou non entérosoluble), une suspension, une dispersion, granules, une poudre, une solution, une émulsion, comprimés à croquer, comprimés pour la dissolution, gouttes, un gel, une pâte, un sirop, une crème, une pastille (poudre, granulés, comprimés), un fluide d'instillation, un gaz, une vapeur, une pommade, un poussoir, implants (oreille, oeil, peau, nez, rectaux ou vaginaux), préparations intramammaires, vagitoires, suppositoires ou utérines.

18. Vaccin selon l'une quelconque des revendications 11 à 17 pour l'administration orale, nasale, vaginale, sublinguale, oculaire, rectale, d'urinal, intramammaire, pulmonaire, auriculaire ou buccale.

19. Vaccin selon l'une quelconque des revendications 11 à 18, dans laquelle la membrane muqueuse est une membrane muqueuse nasale, membrane muqueuse buccale, une membrane muqueuse sublinguale ou une membrane muqueuse gastro-intestinale.

20. Vaccin selon l'une quelconque des revendications 11 à 19 pour l'usage dans le traitement, la prévention ou l'allégement des réactions allergiques.

21. Usage d'un système d'administration muqueuse pour la préparation d'un médicament contenant une substance immunogène dérivée d'allergènes d'inhalation ou dérivée d'allergènes de venin pour l'administration par l'intermédiaire d'une membrane muqueuse d'une vertèbre, dans lequel ledit système d'administration comprend au moins un sel métallique oxygéné sélectionné parmi le groupe composé de l'hydroxyde d'aluminium, du phosphate d'aluminium, du sulfate d'aluminium, de l'acétate d'aluminium, du sulfate d'aluminium de potassium, du phosphate de calcium, du tartrate de calcium, du Maalox (mélange d'hydroxyde d'aluminium et d'hydroxyde de magnésium), de l'hydroxyde de béryllium, de l'hydroxyde de zinc, du carbonate de zinc, du sulfate de baryum et du sulfate de zinc.

22. Usage selon la revendication 21, dans lequel le sel métallique oxygéné est l'hydroxyde d'aluminium, l'acétate d'aluminium, le phosphate d'aluminium, le phosphate de calcium, le tartrate de calcium ou le sulfate de zinc.

23. Usage selon les revendications 21 à 22, dans lequel la substance immunogène est dérivée d'allergènes d'inhalation.

24. Usage selon la revendication 23, dans lequel l'allergène d'inhalation dérive de bois, herbes, verdures, champignons, acariens de poussière de maison, cafards, poils d'animaux et écailles.

25. Usage selon les revendications 21 à 24, comportant en outre un bioadhésif.

26. Usage selon les revendications 21 à 25, dans lequel ledit système d'administration en outre comprend un adjuvant pharmaceutiquement acceptable tel que les interleukines (p. ex. IL-1β, IL-2, IL-7, IL-12, et INFγ), glucane, formulation d'antigène, l'holotoxine du choléra, liposomes, DDE, DHEA, DMPC, DMPG, DOC/Alum Complexe, l'adjuvant incomplet de Freund, adjuvant oral LT, muramyldipeptide, monophosphoryl lipide A, muramyltripeptide et phosphatidyléthanolamine.

27. Usage selon les revendications 21 à 26, dans lequel ledit système d'administration est dans la forme d'un atomiseur, un aérosol, un mélange, des comprimés (à enrobage entérosoluble ou non entérosoluble), capsule (dur ou mou, à enrobage entérosoluble ou non entérosoluble), une suspension, une dispersion, granules, une poudre, une solution, une émulsion, comprimés à croquer, comprimés pour la dissolution, gouttes, un gel, une pâte, un sirop, une crème, une pastille (poudre, granulés, comprimés), un fluide d'instillation, un gaz, une vapeur, une pommade, un poussoir, implants (oreille, oeil, peau, nez, rectaux ou vaginaux), préparations intramammaires, vagitoires, suppositoires ou utérines.

28. Usage selon les revendications 21 à 27, dans lequel ledit système d'administration est formulé pour l'administration orale, nasale, vaginale, sublinguale, oculaire, rectale, d'urinal, intramammaire, pulmonaire, auriculaire ou buccale.

29. Usage selon les revendications 21 à 28, dans lequel la membrane muqueuse est une membrane muqueuse nasale, membrane muqueuse buccale, une membrane muqueuse sublinguale ou une membrane muqueuse gastro-intestinale.

30. Usage selon les revendications 21 à 29 pour la préparation d'un médicament pour le traitement, la prévention ou l'allégement des réactions allergiques.

31. Usage d'une composition selon les revendications 1 à 10 pour la préparation d'un médicament pour le traitement, la prévention ou l'allégement des réactions allergiques.

32. Usage d'un vaccin selon les revendications 11 à 20 pour la préparation d'un médicament pour le traitement, la prévention ou l'allégement des réactions allergiques.

33. Usage selon les revendications 31 à 32 pour la préparation d'un médicament dans une forme pour administration par l'intermédiaire d'une membrane buccale, sublinguale, nasale ou intestinale.

34. Usage selon la revendication 31, dans lequel une composition selon les revendications 1 à 10 est utilisée pour la préparation du vaccin.

35. Usage selon les revendications 31 à 34 pour la préparation d'un vaccin pour le traitement ou la prévention spécifique d'allergie par vaccination.
